# EUROPÄISCHE PATENTSCHRIFT

(11) **EP 1 594 861 B1**
(45) Veröffentlichungstag und Bekanntmachung des Hinweises auf die Patenterteilung: **18.06.2008**
(21) Anmeldenummer: 04708353.0
(22) Anmeldetag: 05.02.2004
(51) Int. Cl.: C07D 403/12, C07D 401/12, A61K 31/41, A61P 25/00

(54) **TRIAZOLVERBINDUNGEN UND IHRE THERAPEUTISCHE VERWENDUNG**
TRIAZOLE COMPOUNDS AND THE THERAPEUTIC USE THEREOF
COMPOSES DE TRIAZOLE ET UTILISATION THERAPEUTIQUE DESDITS COMPOSES

(30) Priorität: 06.02.2003 DE 10304870
(43) Veröffentlichungstag der Anmeldung: 16.11.2005
(73) Patentinhaber: Abbott GmbH & Co. KG, 65205 Wiesbaden (DE)
(72) Erfinder: STARCK, Dorothea, 67292 Kirchheimbolanden (DE); TREIBER, Hans-Jörg, 68782 Brühl (DE); ZIERKE, Thomas, 67459 Böhl-Iggelheim (DE); KETTSCHAU, Georg, 13187 Berlin (DE); GENESTE, Hervé, 67141 Neuhofen (DE); HAUPT, Andreas, 68723 Schwetzingen (DE); UNGER, Liliane, 67065 Ludwigshafen (DE); BLUMBACH, Kai, 79585 Steinen (DE); SCHÖBEL, Dietmar, 68167 Mannheim (DE); TESCHENDORF, Hans-Jürgen, 67373 Dudenhofen (DE)
(74) Vertreter: Pohl, Michael Friedrich
(86) Internationale Anmeldenummer: PCT/EP2004/001072
(87) Internationale Veröffentlichungsnummer: WO 2004/069830

(56) Entgegenhaltungen:
- WO-A-00/42036
- WO-A-97/25324
- WO-A-99/02503
- DE-A- 4 425 144

## Beschreibung

Die vorliegende Erfindung betrifft Triazolverbindungen und ihre therapeutische Verwendung. Die Verbindungen besitzen wertvolle therapeutische Eigenschaften und sind insbesondere zur Behandlung von Erkrankungen geeignet, die auf die Modulation des Dopamin-D₃-Rezeptors ansprechen.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin. Es liegen gesicherte Erkenntnisse über die Anwesenheit von Dopamin und dessen physiologische Funktion als Neurotransmitter vor. Störungen im dopaminergen Transmittersystem resultieren in Erkrankungen des zentralen Nervensystems, zu denen z. B. Schizophrenie, Depression oder Parkinson-Krankheit zählen. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren. In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514 N. Joyce "Dopamine D3-Receptors as a Therapeutic Target for Antipsychotic and Antiparkinsonian Drugs" Pharmacology and Therapeutics, 2001, 90, S. 231-259).

Mittlerweile teilt man die Dopamin-Rezeptoren in zwei Familien ein, einerseits die D₂-Gruppe, bestehend aus D₂-, D₃- und D₄-Rezeptoren, andererseits die D₁-Gruppe, bestehend aus D₁- und D₅-Rezeptoren. Während D₁- und D₂-Rezeptoren weit verbreitet sind, scheinen D₃-Rezeptoren hingegen regioselektiv exprimiert zu werden. So findet man diese Rezeptoren vorzugsweise im limbischen System, den Projektionsbereichen des mesolimbischen Dopaminsystems, vor allem im Nucleus accumbens, aber auch in anderen Bereichen, wie der Amygdala. Wegen dieser vergleichsweise regioselektiven Expression gelten D₃-Rezeptoren als nebenwirkungsarmes Target, und es wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Triazolverbindungen mit Dopamin-D3-Rezeptoraffinität sind aus der DE 4425144, der WO 97/25324 und der WO 99/02503 bekannt.

Weiterhin sind aus der WO 00/42036 Triazolverbindungen der allgemeinen Formel 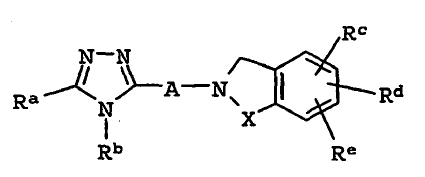 bekannt, worin
- A: für C₄-C₁₀-Alkylen oder für C₃-C₁₀-Alkylen, das wenigstens eine Gruppe Z umfasst, die ausgewählt ist unter O, S, NR', CONR', COO, CO, C₃-C₆-Cycloalkylen und einer Doppel- oder Dreifachbindung, steht, wobei R' z.B. für H oder C₁-C₆-Alkyl steht, das gegebenenfalls durch OH, C₁-C₄-Alkoxy, Halogen oder Phenyl substituiert ist,
- X: für CH₂ oder CH₂-CH₂ steht
- R^{a}: unter anderem ein gegebenenfalls substituierter aromatischer oder heteroaromatischer Rest sein kann,
- R^{b}: H, C₁-C₆-Alkyl, das gegebenenfalls durch OH, C₁-C₄-Alkoxy, Halogen oder Phenyl substituiert ist, C₃-C₆-Cycloalkyl oder Phenyl bedeutet;
- R^{c}, R^{d} und R^{e}: unabhängig voneinander für H, gegebenenfalls substituiertes Alkyl, OH, Alkoxy, Trifluormethoxy, OSO₂CF₃, SH, Alkylthio, Alkenyl, Alkinyl, Halogen, CN, NO₂, CO₂R', SO₂R', SO₂NR'R", CONR'R", NHSO₂R', NR'R", einen carbocylischen 5- oder 6-gliedrigen, gegebenenfalls substituierten aromatischen oder nichtaromatischen Ring oder einen heterocyclischen 5- oder 6-gliedrigen, gegebenenfalls substituierten aromatischen oder nichtaromatischen Ring stehen, wobei R' die zuvor genannten Bedeutungen besitzt, R" die für R' genannen Bedeutungen aufweist oder R' und R" gemeinsam mit dem Stickstoffatom einen 5- bis 7-gliedrigen Stickstoffheterocyclus bilden.

Die aus der WO 00/42036 bekannten Triazolverbindungen weisen eine hohe Affinität gegenüber Dopamin-D₃-Rezeptoren bei einer gleichzeitig hohen Selektivität gegenüber anderen Dopamin-Rezeptoren auf.

Es ist wünschenswert, über selektive Dopamin-D₃-Rezeptor-Liganden zu verfügen, die zudem eine hohe Bioverfügbarkeit, sowie eine hohe Zerebralverfügbarkeit, aufweisen. Verbindungen mit hoher Bioverfügbarkeit weisen den Vorteil auf, dass eine gegebene Schwellenkonzentration des Arzneimittels am Wirkort mit einer niedrigeren oral zu verabreichenden Dosis erreicht werden kann. Zudem wird bei Verabreichung einer gegebenen Dosis eine höhere Konzentration des Arzneimittels am Wirkort erreicht.

Der Erfindung liegt daher die Aufgabe zu Grunde, Verbindungen zur Verfügung zu stellen, die als selektive Dopamin-D₃-Rezeptor-Liganden wirken. Die Verbindungen sollten zudem eine hohe Bioverfügbarkeit, sowie eine hohe Zerebralverfügbarkeit aufweisen.

Diese Aufgabe wird überraschenderweise gelöst durch Triazolverbindungen der allgemeinen Formel I: 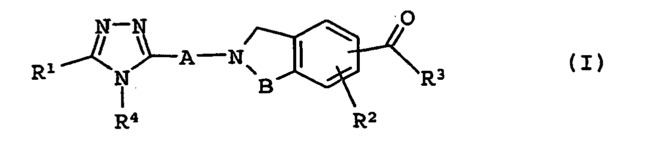 worin
- A: für C₄-C₁₀-Alkylen oder für C₃-C₁₀-Alkylen steht, das wenigstens eine Gruppe Z umfasst, die ausgewählt ist unter O, S, NR⁵, CONR⁵. COO und CO, wobei Alkylen auch eine C₃-C₆-Cycloalkylengruppe und/oder eine Doppel- oder Dreifachbindung aufweisen kann,
- B: für CH₂ oder CH₂-CH₂ steht;
- R¹: für einen aromatischen Rest steht, der ausgewählt ist unter Phenyl und einem 5- oder 6-gliedrigen heteroaromatischen Rest mit 1, 2, 3 oder 4 Heteroatomen, die unabhängig voneinander ausgewählt sind unter O, N und S, wobei der aromatische Rest einen oder mehrere, z.B. 1, 2 oder 3 Substituenten aufweisen kann, die unabhängig voneinander ausgewählt sind unter C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₃-C₆-Cycloalkyl, das gegebenenfalls durch Halogen oder C₁-C₄-Alkyl substituiert ist, Halogen, CN, OR⁶, COOR⁶. NR⁷R⁸, NO₂, SR⁹, SO₂R⁹, SO₂NR⁷R⁸, COR¹⁰, und Phenyl, das gegebenenfalls durch ein oder zwei Reste substituiert ist, die unabhängig voneinander ausgewählt sind unter C₁-C₄-Alkyl, C₁-C₄-Alkoxy, NR⁷R⁸, CN, CF₃, CHF₂ oder Halogen, wobei Phenyl und der heteroaromatische Rest auch mit einem 5 oder 6-gliedrigen, aromatischen oder nichtaromatischen Carbocyclus kondensiert sein kann;
- R²: H, C₁-C₆-Alkyl, das gegebenenfalls durch OH, C₁-C₄-Alkoxy, C₁-C₄-Alkylthio, Halogen oder Phenyl substituiert ist, OH, C₁-C₆-Alkoxy, OCF₃, OCHF₂, OSO₂CF₃, SH, C₁-C₆-Alkylthio, C₂-C₆-Alkenyl, C₂-C₆-Alkinyl, Halogen, CN oder NO₂, bedeutet;
- R³: für C₂-C₁₀-Alkyl, C₁-C₆-Halogenalkyl, C₁-C₁₀-Alkyl, das durch C₁-C₄-Alkoxy, C₁-C₄-Alkylthio oder Phenyl substituiert ist, das seinerseits einen, zwei oder drei Substituenten, ausgewählt unter C₁-C₄-Alkyl, C₁-C₄-Halogenalkyl, Halogen, CN, OR⁶, COOR⁶, NR⁷R⁸, NO₂, SR⁹, SO₂R⁹, SO₂NR⁷R⁸, COR'° und Halogen, aufweisen kann, für C₃-C₆-Cycloalkyl, das gegebenenfalls durch Halogen oder C₁-C₄-Alkyl substituiert ist, oder für einen aromatischen Rest steht, der ausgewählt ist unter Phenyl, Naphthyl und einem 5- oder 6-gliedrigen heteroaromatischen Rest mit 1, 2, oder 3 Heteroatomen, die unabhängig voneinander ausgewählt sind unter O, N und S, wobei der aromatische Rest einen oder zwei Substituenten aufweisen kann, die unabhängig voneinander ausgewählt sind unter C₁-C₆-Alkyl, C₁-C₆-Halogenalkyl, C₁-C₆-Alkoxy, Halogen, CN, COOR⁶, NR⁷R⁸, NO₂, SO₂R⁹, SO₂NR⁷R⁸, COR¹⁰, CF₃, CHF₂ oder Halogen, wobei R³ auch für Methyl stehen kann, wenn R¹ für einen von Phenyl verschiedenen, insbesondere für einen gegebenenfalls substituierten heteroaromatischen Rest steht;
- R⁴: für H, C₁-C₆-Alkyl, das gegebenenfalls durch OH, C₁-C₄-Alkoxy oder Phenyl substituiert ist, C₁-C₆-Halogenalkyl, C₃-C₆-Cycloalkyl, das gegebenenfalls durch C₁-C₄-Alkyl, oder Halogen substituiert ist, oder Phenyl steht;
- R⁵: für H, C₁-C₆-Alkyl, das gegebenenfalls durch OH, C₁-C₄-Alkoxy oder Phenyl substituiert ist, C₁-C₆-Halogenalkyl, Phenyl oder eine Gruppe COR¹¹ steht;
- R⁶ bis R¹⁰: unabhängig voneinander für H, C₁-C₆-Alkyl, das gegebenenfalls durch OH, C₁-C₄-Alkoxy oder Phenyl substituiert ist, C₁-C₆-Halogenalkyl oder Phenyl, stehen, wobei R⁸ auch eine Gruppe COR¹¹ bedeuten kann, worin R¹¹ eine der für R⁴ genannten Bedeutungen aufweist;
- R⁷ mit R⁸: auch gemeinsam einen 5- oder 6-gliedrigen gesättigten oder ungesättigten Carbocyclus bilden kann, der ein Heteroatom, ausgewählt unter O, S, N und NR¹² als Ringglied aufweisen kann, wobei R¹² für Wasserstoff oder C₁-C₄-Alkyl steht,
und durch die physiologisch verträglichen Salze dieser Verbindungen.

Die erfindungsgemäßen Verbindungen der Formel I sind selektive Dopamin-D₃-Rezeptorliganden. Sie eignen sich daher zur Behandlung von Erkrankungen, die auf eine Beeinflussung, d. h. Modulation von derartigen Liganden ansprechen. Beispiele für derartige Erkrankungen sind Störungen und Erkrankungen des zentralen Nervensystems, insbesondere affektive Störungen, neurotische Störungen, Belastungs- und somatoforme Störungen und Psychosen, speziell Schizophrenie und Depression, und weiterhin Nierenfunktionsstörungen, insbesondere solche, die durch Diabetes mellitus hervorgerufen werden (siehe WO 00/67847).

Gegenstand der Erfindung sind daher die Verbindungen I und ihre die Verwendung zur Behandlung derartiger Erkrankungen, bzw. zur Herstellung eines Medikamentes zur Behandlung derartiger Erkrankungen.

Gegenstand der Erfindung ist auch ein pharmazeutisches Mittel (= Medikament), enthaltend wenigstens eine Verbindung der Formel I, gegebenenfalls in Form eines physiologisch verträglichen Salzes von I, und gegebenenfalls einen oder mehrere physiologisch verträgliche Träger und/oder Hilfsstoffe.

Erfindungsgemäß verwendet man zur Behandlung der vorstehend genannten Indikationen wenigstens eine Verbindung der allgemeinen Formel I mit den eingangs genannten Bedeutungen. Sofern die Verbindungen der Formel I ein oder mehrere Asymmetriezentren aufweisen, können auch Enantiomerengemische, insbesondere Racemate, Diastereomerengemische, Tautomerengemische, vorzugsweise jedoch die jeweiligen im Wesentlichen reinen Enantiomere, Diastereomere und Tautomere eingesetzt werden.

Ebenfalls brauchbar sind physiologisch verträgliche Salze der Verbindungen der Formel 1, vor allem Säureadditionssalze mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Cₙ-Cₘ-Alkyl (auch in Resten wie Alkoxy, Alkylthio, Alkylamino etc.) bedeutet eine geradkettige oder verzweigte Alkylgruppe mit n bis m Kohlenstoffatomen, z.B. 1 bis 6 Kohlenstoffatomen und insbesondere 1 bis 4 Kohlenstoffatomen. Beispiele für eine Alkylgruppe sind Methyl, Ethyl, n-Propyl, iso-Propyl, n-Butyl, 2-Butyl, iso-Butyl, tert-Butyl, n-Pentyl, 2-Pentyl, Neopentyl n-Hexyl und dergleichen.

Die Alkylgruppe kann einen oder mehrere Substituenten aufweisen, die unabhängig voneinander ausgewählt sind unter OH, C₁-C₄-Alkoxy, Halogen oder Phenyl. Im Falle eines Halogensubstituenten kann die Alkylgruppe insbesondere 1, 2, 3 oder 4 Halogenatome umfassen, die sich an einem oder mehreren C-Atomen befinden können, vorzugsweise in α- oder ω-Position. Derartige Gruppen werden im Folgenden auch als Halogenalkyl bezeichnet. Bevorzugtes Halogenalkyl ist Fluoralkyl oder Fluorchloralkyl insbesondere CF₃, CHF₂, CF₂Cl, CH₂F, CH₂CF₃.

Im Falle von Hydroxy substituiertem Alkyl weist die Alkylgruppe insbesondere eine Hydroxy-Gruppe auf wie z.B. Hydroxymethyl, 2-Hydroxyeth-1-yl, 2-Hydroxyprop-1-yl, 3-Hydroxy-prop-1-yl, 1-Hydroxyprop-2-yl, 2-Hydroxybut-1-yl, 3-Hydroxybut-1-yl, 4-Hydroxybut-1-yl, 1-Hydroxybut-2-yl, 1-Hydroxybut-3-yl, 2-Hydroxybut-3-yl, 1-Hydroxy-2-methylprop-3-yl, 2-Hydroxy-2-methylprop-3-yl oder 2-Hydroxymethylprop-2-yl, insbesondere 2-Hydroxyethyl.

Im Falle von Alkoxy substituiertem Alkyl weist die Alkylgruppe insbesondere einen Alkoxysubstituenten auf. Diese Reste werden, abhängig von der Anzahl der Kohlenstoffatome auch als Cₙ-Cₘ-Alkoxy-Cₙ-Cₘ-alkyl bezeichnet und stehen z.B. für Methoxymethyl, Ethoxymethyl, 2-Methoxyethyl, 1-Methoxyethyl, 2-Ethoxyethyl, 1-Ethoxyethyl, n-Propoxymethyl, Isopropoxymethyl, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)/propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)-butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise Methoxymethyl, Ethoxymethyl, 2-Methoxyethyl, 2-Ethoxyethyl, 2-(Methoxy)propyl, 2-(Ethoxy)propyl oder 3-(Methoxy)propyl, 3-(Ethoxy)propyl.

Cycloalkyl steht insbesondere für C₃-C₆-Cycloalkyl, wie Cyclopropyl, Cyclobutyl, Cyclopentyl und Cyclohexyl.

Der Begriff "Alkylen" umfasst grundsätzlich geradkettige oder verzweigte Reste mit vorzugsweise 3 bis 10 und besonders bevorzugt 3 bis 8 Kohlenstoffatomen wie Prop-1,2-ylen, Prop-1,3-ylen, But-1,2-ylen, But-1,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, Hex-1,3-ylen, Hex-2,4-ylen, Hex-1,4-ylen, Hex-1,5-ylen, Hex-1,6-ylen und dergleichen. C₀-Alkylen steht für eine Einfachbindung, C₁-Alkylen für Methylen und C₂-Alkylen für 1,1-Ethylen oder 1,2-Ethylen.

"5- oder 6-gliedrige aromatische heterocyclische Reste", die 1, 2, 3 oder 4 Heteroatome aufweisen, die ausgewählt sind unter O, S und N, umfassen insbesondere Heterocyclen mit 1, 2, 3 oder 4 Stickstoffatomen, Heterocyclen mit 1 Sauerstoff oder 1 Schwefelatom, Heterocyclen mit 1 Sauerstoff und 1 oder 2 Stickstoffatomen sowie Heterocyclen mit 1 Schwefelatom und 1 oder 2 Stickstoffatomen. Hierzu zählen vor allem Pyridinyl, Pyrimidinyl, Pyrazinyl, Pyridazinyl, Triazinyl, Imidazolyl, Pyrrolyl, Pyrazolyl, Thienyl, Furanyl, Oxazolyl, Thiazolyl, Isoxazolyl, Tetrazolyl, Thiadiazolyl und Triazolyl. Diese können an den Stickstoffatomen sowie an den Kohlenstoffatomen 1,2 oder 3 der bei R¹ genannten Substituenten aufweisen. Sofern einer der Substituenten Hydroxy ist, können die Reste auch in einer tautomeren Form mit Carbonylgruppe vorliegen. Beispiele für 5- oder 6-gliedrige heterocyclische Reste, die einen anellierten Carbocyclus aufweisen, bzw. mit diesem kondensiert sind, umfassen Benzofuranyl, Benzthienyl, Indolyl, Benzothiazolyl, Benzoxazolyl, Benzimidazolyl, Benzopyrazolyl, Chinolinyl, Isochinolinyl, Chinoxalinyl, Chinazolinyl, Cinnolinyl, sowie entsprechende teilhydrierte Gruppen.

Beispiele für Phenyl, das einen kondensierten Cabocyclus aufweist, sind vor allem Naphthyl, 5,6,7,8-Tetrahydronapht-(1,2,3 oder 4)-yl, Indanyl und Indenyl.

In Zusammenhang mit der Gruppe A befinden sich die beiden Bindungstellen der Alkylenkette vorzugsweise nicht an dem gleichen Atom sondern bilden, gegebenenfalls zusammen mit der wenigstens einen Gruppe Z, eine wenigstens drei- und vorzugsweise wenigstens viergliedrige Kette, die den Triazolring von dem Stickstoffatome des (teil)gesättigten Stickstoffheterocyclus vorzugsweise durch mindestens 5 Bindungen voneinander trennt. Wenn A keine Gruppe Z aufweist, umfasst A 4 bis 10 Kohlenstoffatome, insbesondere 4 bis 8 Kohlenstoffatome. Die Kette zwischen dem Triazolring und der Gruppe B weist dann mindestens vier Kohlenstoffatome auf. Wenn A wenigstens eine der genannten Gruppen Z aufweist, umfasst A 3 bis 10 Kohlenstoffatome, insbesondere 3 bis 8 Kohlenstoffatome. Außerdem können die gesättigte Bindungen in Alkylen durch ungesättigte Bindungen (Alkenylen; Alkinylen) ersetzt sein. So können sich geradkettige oder verzweigte ungesättigte Reste ergeben, deren Anzahl und Anordnung der Kohlenstoffatome derjenigen der zuvor genannten Alkylenreste entspricht, wobei jedoch eine oder mehrere Einfachbindungen durch entsprechende ungesättigte Doppel- bzw. Dreifachbindungen ersetzt sind. Außerdem kann Alkylen eine C₃-C₆-Cycloalkandiylgruppe, z.B. cis- oder trans-Cyclopropan-1,2-diyl, cis- oder trans-Cyclobutan-1,2-diyl, cis- oder trans-Cyclopentan-1,2-diyl, cis- oder trans-Cyclopentan-1,3-diyl, cis- oder trans-Cyclohexan-1,2-diyl, cis- oder trans-Cyclohexan-1,3-diyll, cis- oder trans-Cyclohexan-1,4-diyl, aufweisen.

Wenn die Alkylengruppe in A wenigstens eine der Gruppen Z umfasst, kann diese in der Alkylenkette an beliebiger Stelle oder vorzugsweise in Position 1 oder 2 der Gruppe A (vom Triazolring her gesehen) angeordnet sein. Insbesondere befindet sich Z in der Position 1, d.h. Z ist an den Triazolring gebunden. Die Reste COO und CONR⁵ sind dabei vorzugsweise so angeordnet, dass die Carbonylgruppe an den Triazolring gebunden ist.

Im Hinblick auf die Verwendung der erfindungsgemäßen Verbindungen als Dopamin-D₃-Rezeptor-Liganden weisen die Variablen A, B, R¹, R², R³ und R⁴ unabhängig voneinander vorzugsweise die nachstehend angegebenen Bedeutungen auf:
- A: steht für C₄-C₁₀-Alkylen oder Z-C₃-C₁₀-Alkylen, wobei Z an den Triazol-Ring gebunden ist und Alkylen eine Doppelbindung aufweisen kann. Z weist hierin die vorstehend genannten Bedeutungen auf und ist insbesondere ausgewählt unter O, S, COO, NR⁵ und CO und speziell unter O und S. Vorzugsweise trennt A den Triazolring von dem Stickstoffatome des (teil)gesättigten Stickstoffheterocyclus durch mindestens 5 Bindungen bzw. 4 Kettenglieder. Insbesondere steht A für Z-CH₂CH₂CH₂, Z-CH₂CH₂CH₂CH₂, Z-CH₂CH=CHCH₂, Z-CH₂C(CH₃)=CHCH₂, Z-CH₂C(=CH₂)CH₂, Z-CH₂CH(CH₃)CH₂, (CH₂)₄, (CH₂)₅, CH₂CH₂CH=CHCH₂, CH₂CH₂C(CH₃)=CHCH₂, CH₂CH₂C(=CH₂)CH₂ oder CH₂CH₂CH(CH₃)CH₂. In besonders bevorzugten Verbindungen der Formel I steht A für S-C₃-C₁₀-Alkylen.
- B: steht für CH₂CH₂.
- R¹: steht in einer bevorzugten Ausführungsform der Erfindung für Phenyl, das unsubstituiert ist oder einen oder zwei Substituenten aufweist, die unabhängig voneinander ausgewählt sind unter C₁-C₆-Alkyl, OR⁹, C₁-C₆-Alkoxy, C₁-C₄-Fluoralkyl, Phenyl, CN und Halogen und/oder mit einem 6-gliedrigen, aromatischen Carbocyclus kondensiert sein kann, insbesondere für unsubstituiertes Phenyl oder 4-Fluorphenyl. In einer anderen bevorzugten Ausführungsform steht R¹ für einen 5- oder 6-gliedrigen bevorzugten Ausführungsform steht R¹ für einen 5- oder 6-gliedrigen heteroaromatischen Rest (= Hetaryl), der 1, 2 oder 3 Heteroatome, ausgewählt unter N, O und S aufweisen kann und der einen kondensierten Benzolring aufweisen kann. Der heteroaromatische Rest kann in der oben genannten Weise substituiert sein. Bevorzugte Substituenten an R¹ sind CN, C₁-C₄-Alkyl, OR⁹, Halogen, C₁-C₄-Fluoralkyl, und Phenyl, insbesondere CN, CH₃, OH, OCH₃, CHF₂, CF₃, Halogen, Phenyl und tert.-Butyl. Insbesondere ist R¹ unsubstituiert oder weist einen oder zwei der zuvor als bevorzugt genannten Substituenten auf, z.B. eine Methylgruppe, eine oder zwei Methoxygruppen. Insbesondere steht R¹ für gegebenenfalls substituiertes Pyrrolyl, Thienyl, Furanyl, Tetrazolyl, Benzothienyl, Indolyl, Benzothiazolyl, Pyridyl oder Pyrazinyl, besonders bevorzugt für gegebenenfalls substituiertes 2- oder 3-Pyrrolyl oder gegebenenfalls substituiertes 2- oder 3-Thienyl. Hierunter bevorzugt sind Verbindungen 1, worin R¹ für Pyrrol-2-yl, 1-(C₁-C₄-Alkyl)pyrrol-2-yl, 1-(C₁-C₄-Alkyl)pyrrol-3-yl, 2-Thienyl, 3-Thienyl, Benzothien-2-yl, Benzothiazol-2-yl, 2-Furyl, 3-Furyl, 2-Pyridinyl, 3-Pyridinyl, 4-Pyridinyl oder 2-Pyrazinyl, insbesondere für 1-(C₁-C₄-Alkyl)pyrrol-2-yl, 1-(C₁-C₄-Alkyl)pyrrol-3-yl, 2-Thienyl oder 3-Thienyl steht. In einer besonders bevorzugten Ausführungsform steht R¹ für Pyrrolyl, insbesondere 2-Pyrrolyl, das unsubstituiert ist oder vorzugsweise 1 oder 2 Substituenten ausgewählt unter C₁-C₄-Alkyl und C₃-C₆-Cycloalkyl aufweist, wobei insbesondere ein Substituent am Stickstoff angeordnet ist. Hierunter besonders bevorzugt sind Verbindungen I mit R¹ = 1-Methylpyrrol-2-yl. In einer anderen besonders bevorzugten Ausführungsform steht R¹ für Phenyl, das gegebenenfalls einen der vorgenannten Gruppen und insbesondere ein Halogenatom als Substituenten trägt. In dieser Ausführungsform steht R¹ insbesondere für Phenyl oder p-Fluorphenyl.
- R²: steht vorzugsweise für Wasserstoff.
- R³: steht für C₂-C₁₀-Alkyl, insbesondere C₂-C₆-Alkyl, C₃-C₆-Cycloalkyl, Benzyl oder Phenyl, wobei Benzyl oder Phenyl am Phenylring unsubstituiert oder in der vorgenannten Weise substituiert sein können, z.B. einen oder zwei Substituenten aufweisen, die unabhängig voneinander ausgewählt sind unter C₁-C₆-Alkyl, C₁-C₆-Alkoxy, Halogen, CN, NO₂, CF₃, CHF₂ oder Halogen. Besonders bevorzugt steht R³ für C₂-C₄-Alkyl, insbesondere Ethyl, n-Propyl, iso-Propyl, n-Butyl, 2-Butyl, tert.-Butyl, weiterhin für Cyclopropyl, Cyclobutyl, Cyclopentyl, Cyclohexyl oder für Phenyl, das unsubstituiert ist oder ein- oder zweifach durch C₁-C₄-Alkyl, C₁-C₄-Alkoxy, CF₃, CHF₂ oder Halogen substituiert sein kann. Wenn R¹ für gegebenenfalls substituiertes Hetaryl steht, dann sind auch solche Verbindungen bevorzugt, in denen R³ für Methyl steht. Ganz besonders bevorzugt steht R³ für C₂-C₄-Alkyl.
- R⁴: ist vorzugsweise von Wasserstoff verschieden und steht insbesondere für C₁-C₄-Alkyl und C₃-C₆-Cycloalkyl, besonders bevorzugt für C₁-C₄-Alkyl und speziell für Methyl.

Im Hinblick auf ihre Verwendung sind solche Verbindungen der Formel I besonders bevorzugt, worin die Variablen A, B, R¹, R², R³ und R⁴ gemeinsam die als bevorzugt angegebenen Bedeutungen aufweisen.

Unter den Verbindung der Formel I sind weiterhin solche Verbindungen bevorzugt, in denen die Gruppe -C(O)R³ in der 3-Position (meta-Position) zur Bindungsstelle von B angeordnet ist. Sofern B für CH₂CH₂ steht, ist dies die 7-Position der 1,2,3,4-Tetrahydroisochinolineinheit. Sofern B für CH₂ steht, ist dies die 6-Position der 1,3-Dihydroisoindoteinheit.

In Gruppen NR⁵ steht R⁵ vorzugsweise für H, C₁-C₄-Alkyl, phenylsubstituiertes C₁-C₄-Alkyl oder COR¹¹. Insbesondere bevorzugt steht NR⁵ für NH, NCH₃, NCOCH₃ oder NCH₂-Phenyl.

In Substituenten OR⁶ steht R⁶ vorzugsweise für H, C₁-C₄-Alkyl, CF₃, CHF₂ oder Phenyl. Insbesondere bevorzugt steht OR⁶ für Methoxy, Trifluormethoxy oder Phenoxy.

In Substituenten COOR⁶ steht R⁶ für H oder C₁-C₄-Alkyl. Insbesondere bevorzugt steht COOR⁶ für C₁-C₄-Alkoxycarbonyl, wie Methoxycarbonyl, Ethoxycarbonyl, n-Propoxycarbonyl, i-Propoxycarbonyl, n-Butoxycarbonyl oder t-Butoxycarbonyl.

In Substituenten CONR⁷R⁸ steht R⁷ vorzugsweise für H oder C₁-C₄-Alkyl und R⁸ vorzugsweise für H, C₁-C₄-Alkyl oder COR¹¹. Insbesondere bevorzugt steht CONR⁷R⁸ für CONH₂, CONHCH₃, CON(CH₃)₂ oder CONHCOCH₃.

In Substituenten NR⁷R⁸ steht R⁷ vorzugsweise für H, C₁-C₄-Alkyl oder phenylsubstituiertes C₁-C₄-Alkyl und R⁸ für H, C₁-C₄-Alkyl oder COR¹¹. Insbesondere bevorzugt steht NR⁷R⁸ für NH₂, NHCH₃, N(CH₃)₂, NH-Benzyl oder NHCOCH₃.

In Substituenten SO₂NR⁷R⁸ steht R⁷ vorzugsweise für H oder C₁-C₄-Alkyl und R⁸ vorzugsweise für H, C₁-C₄-Alkyl oder COR¹¹. Insbesondere bevorzugt steht SO₂NR⁷R⁸ für Sulfamoyl.

In Substituenten SR⁹ steht R⁹ vorzugsweise für Alkyl. Insbesondere bevorzugt steht SR⁹ für Thiomethyl.

In Substituenten SO₂R⁹ steht R⁹ vorzugsweise für H oder C₁-C₄-Alkyl. Insbesondere bevorzugt steht SO₂R⁹ für Methylsulfonyl.

In Substituenten COR¹⁰ steht R¹⁰ vorzugsweise für H, C₁-C₄-Alkyl oder Phenyl. Insbesondere bevorzugt steht COR¹⁰ für Formyl, Acetyl oder Benzoyl.

In Substituenten COR¹¹ steht R¹¹ vorzugsweise für H, C₁-C₄-Alkyl oder Phenyl. Insbesondere bevorzugt steht COR¹¹ für Formyl, Acetyl oder Benzoyl.

Eine bevorzugte Ausführungsform der Erfindung sind die Verbindungen der allgemeinenen Formel Ia, 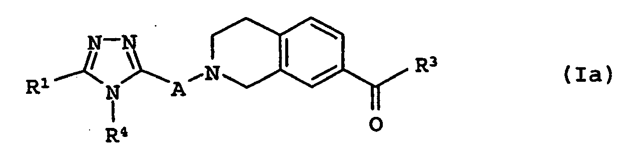 worin A, R¹ R³ und R⁴ die zuvor angegebenen Bedeutungen aufweisen, d.h. Verbindungen der allgemeinen Formell, wie zuvor definiert, in denen B für eine CH₂-CH₂-Einheit steht, R² Wasserstoff bedeutet und der Rest C(O)R³ in der 7-Position angeordnet ist. Hinsichtlich der bevorzugten Bedeutungen für R¹, R³, R⁴ und A gilt das zuvor gesagte.

Insbesondere steht A für C₄-C₁₀-Alkylen oder Z-C₃-C₁₀-Alkylen, wobei Z an den Triazol-Ring gebunden ist und Alkylen eine Doppelbindung aufweisen kann. Z weist hierin die vorstehend genannten Bedeutungen auf und ist insbesondere ausgewählt unter O, S, COO, NR⁵ und CO und speziell unter O und S. Vorzugsweise trennt A den Triazolring von dem Stickstoffatome des (teil)gesättigten Stickstoffheterocyclus durch mindestens 5 Bindungen bzw. 4 Kettenglieder. Insbesondere steht A für Z-CH₂CH₂CH₂, Z-CH₂CH₂CH₂CH₂, Z-CH₂CH=CHCH₂, Z-CH₂C(CH₃)=CHCH₂, Z-CH₂C(=CH₂)CH₂, Z-CH₂CH(CH₃)CH₂, (CH₂)₄, (CH₂)₅, CH₂CH₂CH=CHCH₂, CH₂CH₂C(CH₃)=CHCH₂, CH₂CH₂C(=CH₂)CH₂ oder CH₂CH₂CH(CH₃)CH₂. In besonders bevorzugten Verbindungen der Formel I steht A für S-C₃-C₁₀-Alkylen.

Insbesondere steht R¹ in Formel Ia für gegebenenfalls substituiertes Phenyl, Pyrrolyl, Thienyl, Furanyl, Tetrazolyl, Benzothienyl, Indolyl, Benzothiazolyl, Pyridyl und Pyrazinyl, besonders bevorzugt für gegebenenfalls substituiertes 2- oder 3-Pyrrolyl oder gegebenenfalls substituiertes 2- oder 3-Thienyl. Hierunter bevorzugt sind Verbindungen I, worin R¹ für Pyrrol-2-yl, 1-(C₁-C₄-Alkyl)pyrrol-2-yl, 1-(C₁-C₄-Alkyl)pyrrol-3-yl, 2-Thienyl, 3-Thienyl, Benzothien-2-yl, Benzothiazol-2-yl, 2-Furyl, 3-Furyl, 2-Pyridinyl, 3-Pyridinyl, 4-Pyridinyl oder 2-Pyrazinyl insbesondere für 1-(C₁-C₄-Alkyl)pyrrol-2-yl, 1-(C₁-C₄-Alkyl)pyrrol-3-yl, 2-Thienyl oder 3-Thienyl steht. In einer besonders bevorzugten Ausführungsform steht R¹ für Pyrrolyl, insbesondere 2-Pyrrolyl, das unsubstituiert ist oder vorzugsweise 1 oder 2 Substituenten ausgewählt unter C₁-C₄-Alkyl und C₃-C₆-Cycloalkyl aufweist, wobei insbesondere ein Substituent am Stickstoff angeordnet ist. Hierunter besonders bevorzugt sind Verbindungen I mit R¹ = 1-Methylpyrrol-2-yl. In einer anderen besonders bevorzugten Ausführungsform steht R¹ für Phenyl, das gegebenenfalls einen der vorgenannten Gruppen und insbesondere ein Halogenatom als Substituenten trägt. In dieser Ausführungsform steht R¹ insbesondere für Phenyl oder p-Fluorphenyl.

In Formel la steht R³ insbesondere für C₂-C₆-Alkyl, C₃-C₆-Cycloalkyl, Benzyl oder Phenyl, wobei Benzyl oder Phenyl am Phenylring unsubstituiert oder in der vorgenannten Weise substituiert sein können, z.B. einen oder zwei Substituenten aufweisen, die unabhängig voneinander ausgewählt sind unter C₁-C₆-Alkyl, C₁-C₆-Alkoxy, Halogen, CN, NO₂, CF₃, CHF₂ oder Halogen. Besonders bevorzugt steht R³ für C₂-C₄-Alkyl, insbesondere Ethyl, n-Propyl, iso-Propyl, n-Butyl, 2-Butyl, tert.-Butyl, weiterhin Cyclopropyl, Cyclobutyl, Cyclopentyl, Cyclohexyl oder Phenyl, das unsubstituiert ist oder ein- oder zweifach durch C₁-C₄-Alkyl, C₁-C₄-Alkoxy, CF₃, CHF₂ oder Halogen substituiert sein kann. Wenn R¹ für gegebenenfalls substituiertes Hetaryl steht, dann sind auch solche Verbindungen bevorzugt, in denen R³ für Methyl steht. Ganz besonders bevorzugt steht R³ für C₂-C₄-Alkyl.

In Formel Ia steht R⁴ vorzugsweise für einen von Wasserstoff verschiedenen Rest und insbesondere für C₁-C₄-Alkyl und C₃-C₆-Cycloalkyl, insbesondere für C₁-C₄-Alkyl und speziell für Methyl.

Unter den Verbindungen der Formel Ia sind insbesondere Verbindungen der Formel Ia' 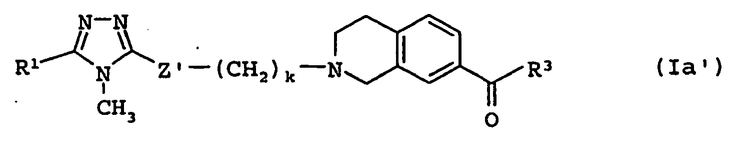 bevorzugt, worin R¹ und R³ die zuvor angegebenen, insbesondere die als bevorzugt angegebenen Bedeutungen aufweisen, k für 3 oder 4 und Z' für O, CH₂ oder insbesondere für S stehen.

Hierunter sind insbesondere solche Verbindungen der Formel Ia' bevorzugt, worin R¹ und R³ alleine oder besonders bevorzugt in Kombination die folgenden Bedeutungen aufweisen:
- R¹: Phenyl, 4-Fluorphenyl, Pyrrol-2-yl, 1-Methylpyrrol-2-yl, 2-Thienyl, 3-Thienyl, Benzothien-2-yl, 2-Furyl, 3-Furyl, Benzothiazol-2-yl, 2-Pyridinyl, 3-Pyridinyl, 4-Pyridinyl oder 2-Pyrazinyl. Insbesondere steht R¹ für 1-Methylpyrrol-2-yl;
- R³: C₂-C₄-Alkyl oder Phenyl, das unsubstituiert ist oder ein- oder zweifach durch C₁-C₄Alkyl, C₁-C₄-Alkoxy, CF₃, CHF₂ oder Halogen substituiert sein kann, oder wenn R¹ von Phenyl verschieden ist auch Methyl bedeuten kann. Insbesondere steht R³ für C₂-C₄-Alkyl.

Beispiele für besonders bevorzugte Verbindungen 1 sind in der nachfolgenden Tabelle 1 aufgeführten Verbindungen der Formel Ia', worin R¹, R³, k und Z' in Formel Ia' die in jeweils einer Zeile der Tabelle 1 angegebene Bedeutung aufweisen. (Verbindungen Ia'-1 bis 1a'-92).

**Tabelle 1:**

| Verbindung Ia' Nr. | R¹ | Z' | k | R³ |
|---|---|---|---|---|
| 1 | 4-Pyridyl | S | 3 | Phenyl |
| 2 | 3-Thienyl | S | 3 | tert.-Butyl |
| 3 | 2-Furyl | S | 3 | Phenyl |
| 4 | 1-Methylpyrrol-2-yl | S | 3 | Methyl |
| 5 | 2-Thienyl | S | 3 | Ethyl |
| 6 | 3-Thienyl | S | 3 | Methyl |
| 7 | Phenyl | S | 3 | Cyclohexyl |
| 8 | 3-Pyridyl | S | 3 | Cyclopropyl |
| 9 | 4-Pyridyl | S | 3 | Ethyl |
| 10 | 3-Pyridyl | S | 3 | tert.-Butyl |
| 11 | 4-Pyridyl | S | 3 | Cyclohexyl |
| 12 | 1-Methylpyrrol-2-yl | S | 3 | Phenyl |
| 13 | Phenyl | O | 3 | Phenyl |
| 14 | 3-Thienyl | S | 3 | Cyclopropyl |
| 15 | 1-Methylpyrrol-2-yl | S | 3 | Ethyl |
| 16 | 2-Furyl | S | 3 | Cyclohexyl |
| 17 | Phenyl | CH₂ | 3 | Phenyl |
| 18 | 3-Thienyl | S | 3 | Phenyl |
| 19 | Phenyl | S | 3 | Phenyl |
| 20 | 3-Thienyl | S | 3 | Ethyl |
| 21 | Phenyl | CH₂ | 3 | Ethyl |
| 22 | 2-Pyrazinyl | S | 3 | Cyclopropyl |
| 23 | 1-Methylpyrrol-2-yl | S | 3 | Cyclopropyl |
| 24 | 3-Thienyl | S | 3 | Cyclohexyl |
| 25 | Phenyl | S | 3 | Ethyl |
| 26 | 2-Pyrazinyl | S | 3 | Ethyl |
| 27 | 1-Methylpyrrol-2-yl | O | 3 | Ethyl |
| 28 | 2-Pyrazinyl | S | 3 | Methyl |
| 29 | 4-Pyridyl | S | 3 | tert.-Butyl |
| 30 | 2-Furyl | S | 3 | Methyl |
| 31 | 1-Methylpyrrol-2-yl | S | 3 | tert.-Butyl |
| 32 | 2-Furyl | S | 3 | Ethyl |
| 33 | 4-Pyridyl | S | 3 | Methyl |
| 34 | Phenyl | S | 3 | Cyclopropyl |
| 35 | Phenyl | S | 3 | tert.-Butyl |
| 36 | 2-Thienyl | S | 3 | Methyl |
| 37 | 2-Pyrazinyl | S | 3 | Phenyl |
| 38 | 2-Thienyl | S | 3 | Phenyl |
| 39 | 3-Pyridyl | S | 3 | Methyl |
| 40 | 1-Methylpyrrol-2-yl | CH₂ | 3 | Ethyl |
| 41 | 3-Pyridyl | S | 3 | Ethyl |
| 42 | 2-Thienyl | S | 3 | Cyclohexyl |
| 43 | 2-Furyl | S | 3 | Cyclopropyl |
| 44 | 2-Pyrazinyl | S | 3 | Cyclohexyl |
| 45 | 4-Pyridyl | S | 3 | Cyclopropyl |
| 46 | 3-Pyridyl | S | 3 | Phenyl |
| 47 | 1-Methylpyrrol-2-yl | S | 4 | Ethyl |
| 48 | 2-Thienyl | S | 3 | Cyclopropyl |
| 49 | Phenyl | O | 3 | Ethyl |
| 50 | 2-Pyrazinyl | S | 3 | tert.-Butyl |
| 51 | 3-Pyridyl | S | 3 | Cyclohexyl |
| 52 | 2-Thienyl | S | 3 | tert.-Butyl |
| 53 | 1-Methylpyrrol-2-yl | S | 3 | Cyclohexyl |
| 54 | 2-Furyl | S | 3 | tert.-Butyl |
| 55 | 2-Thienyl | S | 3 | 3-Fluorphenyl |
| 56 | Phenyl | S | 4 | 3-Fluorphenyl |
| 57 | Benzo[b]thien-2-yl | S | 3 | Ethyl |
| 58 | 2-Pyrazinyl | S | 3 | 3-Fluorphenyl |
| 59 | 1H-Pyrrol-2-yl | S | 3 | tert.-Butyl |
| 60 | 1-Methylpyrrol-2-yl | S | 3 | 3-Fluorphenyl |
| 61 | Benzo[b]thiazol-2-yl | S | 3 | Phenyl |
| 62 | 2-Furyl | S | 3 | 3-Fluorphenyl |
| 63 | 3-Pyridyl | S | 3 | 3-Fluorphenyl |
| 64 | 1H-Pyrrol-2-yl | S | 3 | Phenyl |
| 65 | 1H-Pyrrol-2-yl | S | 3 | Ethyl |
| 66 | 3-Thienyl | S | 3 | 3-Fluorphenyl |
| 67 | Phenyl | S | 4 | Phenyl |
| 68 | Phenyl | S | 3 | 3-Fluorphenyl |
| 69 | 1-Methylpyrrol-2-yl | S | 3 | 3-Fluorphenyl |
| 70 | Benzo[b]thiazol-2-yl | S | 3 | Ethyl |
| 71 | Pyridin-2-yl | S | 3 | Ethyl |
| 72 | 1-Methylpyrrol-2-yl | S | 4 | 3-Fluorphenyl |
| 73 | 1H-Pyrrol-2-yl | S | 3 | Cyclopropyl |
| 74 | 1H-Pyrrol-2-yl | S | 3 | Cylcohexyl |
| 75 | Benzo[b]thien-2-yl | S | 3 | Phenyl |
| 76 | 3-Pyridyl | S | 3 | Ethyl |
| 77 | Benzo[b]thiazol-2-yl | S | 3 | 3-Fluorphenyl |
| 78 | 2-Furyl | S | 4 | Cyclopropyl |
| 79 | 2-Thienyl | S | 4 | Cyclohexyl |
| 80 | Benzo[b]thien-2-yl | S | 3 | Cyclopropyl |
| 81 | 4-F-Phenyl | S | 3 | Ethyl |
| 82 | 4-F-Phenyl | CH₂ | 3 | Ethyl |
| 83 | 2-Thienyl | S | 3 | 4-Fluorphenyl |
| 84 | Phenyl | S | 4 | 4-Fluorphenyl |
| 85 | 2-Pyrazinyl | S | 3 | 4-Fluorphenyl |
| 86 | 1-Methylpyrrol-2-yt | S | 3 | 4-Fluorphenyl |
| 87 | 3-Pyridyl | S | 3 | 4-Fluorphenyl |
| 88 | 3-Thienyl | S | 3 | 4-Fluorphenyl |
| 89 | Phenyl | S | 3 | 4-Fluorphenyl |
| 90 | 1-Methylpyrrol-2-yl | S | 3 | 4-Fluorphenyl |
| 91 | 1-Methylpyrrol-2-yl | S | 4 | 4-Fluorphenyl |
| 92 | Benzo[b]thiazol-2-yl | S | 3 | 4-Fluorphenyl |

Eine andere bevorzugte Ausführungsform der Erfindung sind die Verbindungen der allgemeinenen Formel Ib, 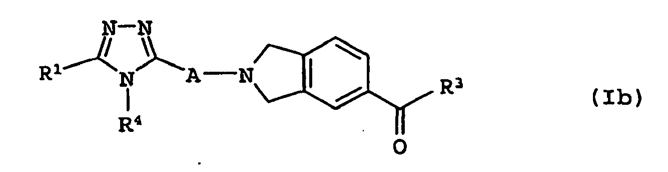 worin A, R¹ R³ und R⁴ die zuvor angegebenen Bedeutungen aufweisen, d.h. Verbindungen der allgemeinen Formel I, wie zuvor definiert, in denen B für eine CH₂-Einheit steht, R² Wasserstoff bedeutet und der Rest C(O)R³ in der 6-Position angeordnet ist. Hinsichtlich der bevorzugten Bedeutungen für R¹, R³, R⁴ und A gilt das zuvor für Formel Ia gesagte.

Unter den Verbindungen der Formel Ib sind insbesondere Verbindungen der Formel Ib' 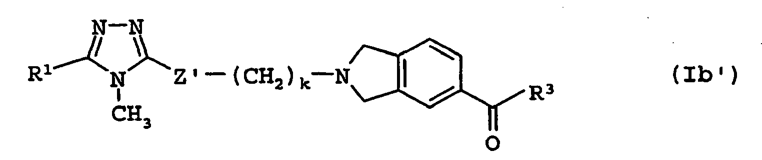 bevorzugt, worin R¹ und R³ die zuvor angegebenen, insbesondere die als bevorzugt angegebenen Bedeutungen aufweisen, k für 3 oder 4 und Z' für O, CH₂ oder insbesondere für S stehen.

Hierunter sind insbesondere solche Verbindungen der Formel Ib' bevorzugt, worin R¹ und R³ alleine oder besonders bevorzugt in Kombination die folgenden Bedeutungen aufweisen:
- R¹: Phenyl, 4-Fluorphenyl, Pyrrol-2-yl, 1-Methylpyrrol-2-yl, 2-Thienyl, 3-Thienyl, Benzothien-2-yl, 2-Furyl, 3-Furyl, Benzothiazol-2-yl, 2-Pyridinyl, 3-Pyridinyl, 4-Pyridinyl oder 2-Pyrazinyl. Insbesondere steht R¹ für 1-Methylpyrrol-2-yl;
- R³: C₂-C₄-Alkyl oder Phenyl, das unsubstituiert ist oder ein- oder zweifach durch C₁-C₄-Alkyl, C₁-C₄-Alkoxy, CF₃, CHF₂ oder Halogen substituiert sein kann, oder wenn R¹ von Phenyl verschieden ist auch Methyl bedeuten kann. Insbesondere steht R³ für C₂-C₄-Alkyl.

Beispiele für besonders bevorzugte Verbindungen Ib sind Verbindungen der Formel Ib', worin R¹, R³, k und Z' in Formel Ib' die in jeweils einer Zeile der Tabelle 1 angegebene Bedeutung aufweisen. (Verbindungen Ib'-1 bis 1b'-92).

Die Herstellung der erfindungsgemäßen Verbindungen erfolgt analog zu literaturbekannten Methoden, die für die Herstellung von Triazolverbindungen dieses Typs z.B. in der WO 00/42036 beschrieben sind. In der Regel wird man hierzu
a) eine Verbindung der allgemeinen Formel (II) 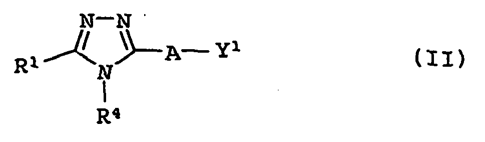 worin Y¹ für eine übliche Abgangsgruppe wie beispielsweise Halogen, Alkylsulfonyloxy, Arylsulfonyloxy, Trifluormethylsulfonyloxy oder dergleichen steht, mit einer Verbindung der allgemeinen Formel (III) 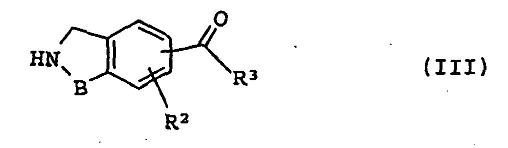 umsetzen; oder
b) eine Verbindung der allgemeinen Formel (IV) 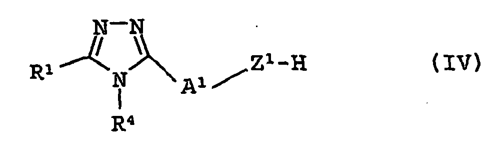 worin Z¹ für O, S oder NR⁵ und A¹ für eine Bindung oder für C₁-C₁₀-Alkylen stehen, mit einer Verbindung der allgemeinen Formel (V) umsetzen, 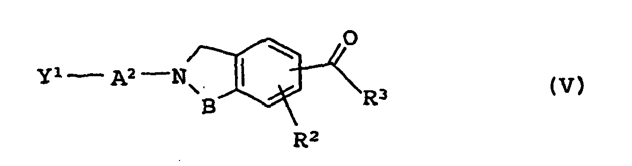 wobei Y¹ die oben angegebene Bedeutung besitzt und A² für C₂-C₁₀-Alkylen steht, wobei A¹ und A² zusammen 3 bis 10 C-Atome aufweisen und A¹ und/oder A² gegebenenfalls wenigstens eine Gruppe Z umfassen; oder
c) eine Verbindung der allgemeinen Formel (VI) 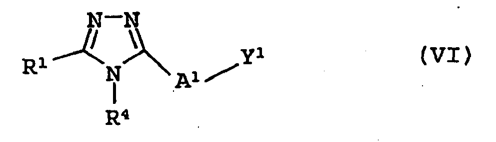 worin Y¹ und A¹ die oben angegebenen Bedeutungen besitzen, mit einer Verbindung der allgemeinen Formel (VII) 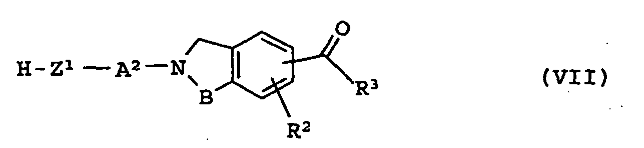 worin Z¹ und A² die oben angegebenen Bedeutungen besitzt, umsetzen; oder
d) einen Aldehyd der allgemeinen Formel (VIII) 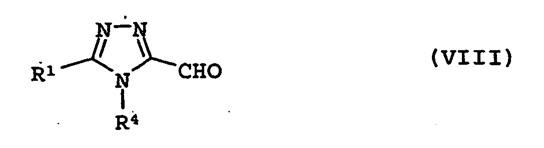 mit literaturbekannten Reagenzien, wie z. B. 1,3-Propandithiol, KCN/Wasser, TMSCN (Trimethylsilylcyanid) oder KCN/Morpholin, wie z. B. beschrieben in
   Albright Tetrahedron, 1983, 39, 3207 oder
   D. Seebach Synthesis 1969, 17 und 1979, 19 oder
   H. Stetter Angew. Chem. Int. Ed. 1976, 15, 639 oder
   van Niel et al. Tetrahedron 1989, 45, 7643
   Martin et al. Synthesis 1979, 633,
   zu den Produkten (VIIIa) (exemplarisch mit 1,3-Propandithiol) 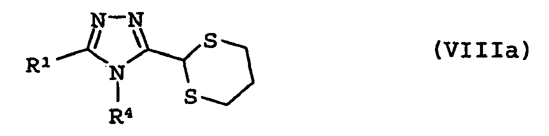 umpolen und anschließend mit Verbindungen der allgemeinen Formel (IX) kettenverlängeren, 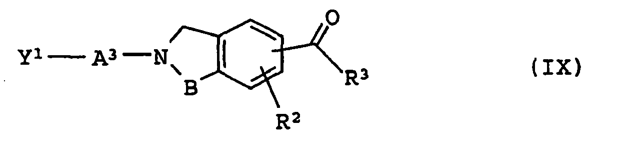 wobei Y¹ die oben angegebene Bedeutung besitzt und A³ für C₃-C₉-Alkylen steht, das eine Gruppe Z enthalten kann. Gegebenenfalls liegt die Carbonylfunktion in der Gruppe -C(O)-R³ in Formel IX in geschützter Form vor. Nach Entschützen oder Reduktion erhält man auf diese Weise Verbindungen der Formel (I-1), 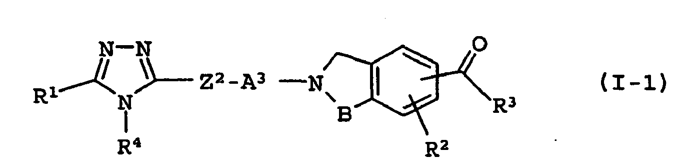 worin Z² für CO oder eine Methylengruppe steht und Z² und A³ zusammen 4 bis 10 C-Atome aufweisen. Alternativ kann man
e) einen Aldehyd der allgemeinen Formel (VIII) mit einer Verbindung der allgemeinen Formel (X) umsetzen, 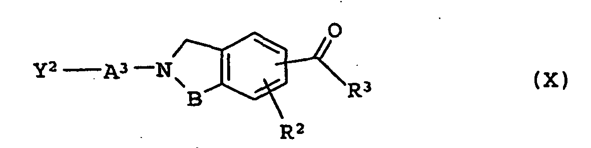 worin Y² für ein Phosphoran oder einen Phosphonsäureester steht und A³ die oben angegebene Bedeutung aufweist, analog zu üblichen Methoden, wie zum Beispiel beschrieben in Houben Weyl "Handbuch der Organischen Chemie" 4. Auflage, Thieme Verlag Stuttgart, Band V/1b S.383 ff oder Bd V/1c S.575 ff, oder
f) eine Verbindung der allgemeinen Formel (XI) 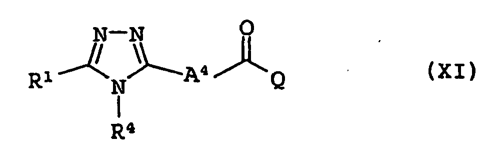 worin A⁴ für C₃-C₉-Alkylen oder C₂C₉-Alkylen, das eine Gruppe Z wie vorstehend definiert umfasst, steht, Q für H oder OH steht, mit einer Verbindung der Formel lila unter reduktiven Bedingungen umsetzen. Die Umsetzung erfolgt analog literaturbekannten Methoden, wie z. B. beschrieben in J. Org. Chem. 1986, 50, 1927 oder WO 92/20655.

Das Verfahren zur Herstellung einer Verbindung der Formel I, worin A die Gruppe COO umfasst, besteht darin, dass man eine Verbindung der allgemeinen Formel XII 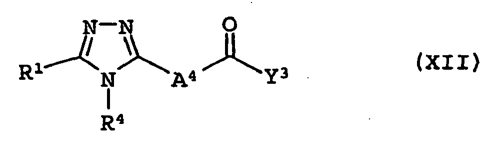 worin Y³ für OH, OC₁-C₄-Alkyl, Cl oder zusammen mit CO für eine aktivierte Carboxylgruppe (Aktivestergruppe), und A⁴ für C₀-C₉-Alkylen steht, mit einer Verbindung der Formel XIII umsetzt 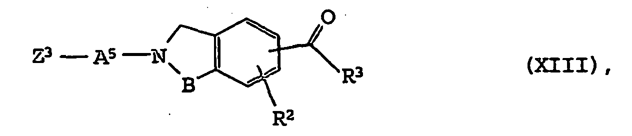 worin Z³ für OH steht und A⁵ für C₂-C₆-Alkylen steht, wobei A⁵ und A⁴ gemeinsam 3 bis 10 C-Atome aufweisen. In analoger Weise können durch Umsetzung von XII mit XIII, worin Z³ für NHR⁵ steht, Verbindungen I mit 2 = CONR⁵ hergestellt werden.

Verbindungen der allgemeinen Formel III sind literaturbekannt oder können hergestellt werden, indem man in eine Verbindung der allgemeinen Formel XIV, 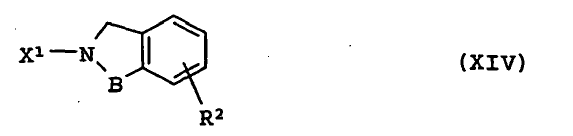 worin X¹ für Wasserstoff oder vorzugsweise eine NH-Schutzgruppe steht, in an sich bekannter Weise eine Gruppe -C(O)-R³ einführt, z.B. in dem man die Verbindung XIV unter Friedel-Crafts-Bedingungen acyliert, wobei man eine Verbindung der Formel IIIa erhält, 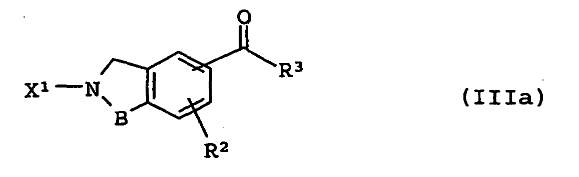 und gegebenenfalls die Schutzgruppe X¹ entfernt. Geeignete NH-Schutzgruppen, ihre Einführung und ihre Entfernung sind z.B. aus Kocienski, "Protecting Groups" Georg Thieme-Verlag, Stuttgart, New-York, 2000, S. 185-216 beschrieben und umfassen insbesondere Trifluormethylcarbonyl, Methoxy- und Ethoxycarbonyl, Boc-, Z-, Zbz-, Aloc-, Fmoc-, Teoc- und Troc-Gruppen.

Geeignete Methoden für die Friedel-Crafts-Acylierung von Verbindungen XIV sind beispielsweise beschrieben in G.L.Grunewald et al, J. Med. Chem. 42 (1999), S. 118-134, F.E. Ali et al., J. Med. Chem. 25 (1982), S. 1235-1240.

Die Herstellung von Verbindungen der Formel III bzw. IIIa gelingt ausserdem durch Cyclisierung von Verbindungen der allgemeinen Formel XV 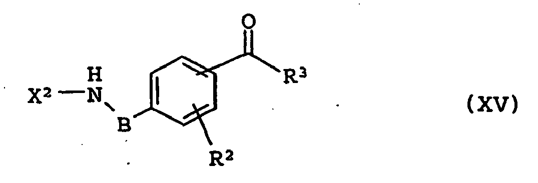 worin X² für eine Alkylcarbonylgruppe, z.B. Acetyl steht, mit Formaldehyd oder einem Formaldehyd-Äquivalent wie Forrnalin-Lösung, Trioxan, Paraformaldehyd und dergleichen unter Säurekatalyse analog dem in A.B. Venkov et al., Synth. Commun. 25 (1995), S. 1419-1425 beschriebenen Verfahren.

Aus den Verbindungen der Formel III bzw. IIIa können die Verbindungen der Formeln V, VII, IX, X und XIII in Analogie zu den in der eingangs zitierten Patentliteratur, z.B. nach den Verfahren der WO 00/42036 hergestellt werden.

In den obigen Formeln II bis XV besitzen R¹, R², R³, R⁴, A, und B und X die im Zusammenhang mit den Formeln I, Ia bzw. Ia' angegebenen Bedeutungen.

Verbindungen der allgemeinen Formel XIV sind literaturbekannt oder können nach den in P. L. Julian, J. Am. Chem. Soc. 70 (1948) S. 180-183, G.E. Hein, J. Am. Chem. Soc. 84 (1962), S. 4487-4494 sowie WO 00/21905 beschriebenen Methoden hergestellt werden.

Die Triazolverbindungen der Formeln II, IV, VI, VIII, VIIIa, XI und XII sind entweder bekannt oder können nach bekannten Verfahren hergestellt werden, wie z. B. beschrieben in A.R. Katritzky, C.W. Rees (ed.) "Comprehensive Heterocyclic Chemistry", Pergamon Press, oder "The Chemistry of Heterocyclic Compounds" J. Wiley & Sons Inc. NY und der dort zitierten Literatur oder in S. Kubota et al. Chem. Pharm. Bull 1975, 23, 955, WO 99/02503 oder Vosilevskii et al. Izv. Akad. Nauk. SSSR Ser. Khim 1975, 23, 955; Rappoport et al. J. Org. Chem. 37 (1972), S. 3618.

Die Herstellung der erfindungsgemäßen Verbindungen und der Ausgangsmaterialien und der Zwischenprodukte kann auch analog zu den in den eingangs genannten Patentpublikationen beschriebenen Methoden erfolgen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ester, wie Ethylacetat, Ether, wie Diethylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Ethanol oder Butanol.

Gewünschtenfalls arbeitet man in Gegenwart eines säurebindenden Mittels. Geeignete säurebindende Mittel sind anorganische Basen, wie Natrium- oder Kaliumcarbonat, Natrium- oder Kaliumhydrogencarbonat, Natriummethylat, Natriumethylat, Natriumhydrid oder metallorganische Verbindungen, wie Butyllithium- oder Alkylmagnesium-Verbindungen, oder organische Basen, wie Triethylamin oder Pyridin. Letztere können gleichzeitig als Lösungsmittel dienen.

Das Verfahren (f) erfolgt unter reduzierenden Bedingungen, z.B. unter Verwendung von Natriumborhydrid, Natriumcyanoborhydrid oder Triacetoxyborhydrid, gegebenenfalls in saurem Medium oder in Gegenwart einer Lewissäure, wie z.B. Zinkchlorid oder mittels katalytischer Hydrierung.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder überführen in eine Säureadditionsverbindung.

Die Säureadditionssalze werden in üblicher Weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Bei den erfindungsgemäßen Triazolverbindungen der Formel handelt es sich um hochselektive Dopamin-D₃-Rezeptor-Liganden, die auf Grund ihrer geringen Affinität gegenüber anderen Rezeptoren wie D₁-Rezeptoren, D₄-Rezeptoren, α1- und/oder α2-adrenergen-Rezeptoren, serotinergen Rezeptoren, muskarinergen Rezeptoren, histaminischen Rezeptoren, Opiat-rezeptoren und insbesondere gegenüber Dopamin-D₂-Rezeptoren, nebenwirkungsärmer als die klassischen Neuroleptika sind, bei denen es sich um D₂-Rezeptor-Antagonisten handelt.

Die hohe Affinität der erfindungsgemäßen Verbindungen gegenüber D₃-Rezeptoren spiegelt sich in sehr geringen in vitro Kᵢ-Werte von in der Regel weniger als 100 nM (nmol/l) und vor allem von weniger als 50 nM wieder. Beispielsweise können Bindungsaffinitäten zu D₃-Rezeptoren in Rezeptorbindungsstudien über die Verdrängung von [¹²⁵I]-Iodosulprid bestimmt werden.

Erfindungsgemäß von besonderer Bedeutung sind Verbindungen deren Selektivität Kᵢ(D₂)/Kᵢ(D₃) vorzugsweise wenigstens 10, besser noch wenigstens 30 und besonders vorteilhaft wenigstens 50 beträgt. Rezeptorbindungsstudien an D₁-, D₂- und D₄-Rezeptoren können beispielsweise über die Verdrängung von [³H]SCH23390, [¹²⁵I]Iodosulprid bzw. [¹²⁵I]Spiperon vorgenommen werden.

Die erfindungsgemäßen Verbindungen weisen ausserdem eine bessere Bioverfügbarkeit sowie eine bessere zerebrale Verfügbarkeit auf als vergleichbare D₃-Rezoptorliganden auf Basis von bekannten Triazolverbindungen mit Tetrahydroisoquinolin- oder Dihydroisoindolrest und sollten daher gegenüber vergleichbaren Substanzen die oben erwähnten Vorteile aufweisen.

Die Verbindungen sind aufgrund ihres Bindungsprofils zur Behandlung von Erkrankungen brauchbar, die auf Dopamin-D₃-Liganden ansprechen, d. h. sie sind zur Behandlung von solchen Störungen bzw. Erkrankungen wirksam, bei denen eine Beeinflussung (Modulation) der Dopamin-D₃-Rezeptoren zur Verbesserung des Krankheitsbilds oder zum Ausheilen der Krankheit führt. Derartige Erkrankungen sind z. B. Störungen oder Erkrankungen des zentralen Nervensystems

Unter Störungen bzw. Erkrankungen des Zentralen Nervensystems versteht man Störungen, die Rückenmark und vor allem das Gehirn betreffen. Der Begriff "Störung" im erfindungsgemäßen Sinne bezeichnet Anomalien, die in der Regel als krankhafte Zustände bzw. Funktionen angesehen werden und sich in Form bestimmter Anzeichen, Symptome und/oder Fehlfunktionen zu erkennen geben können. Die erfindungsgemäße Behandlung kann auf einzelne Störungen, d. h. Anomalien bzw. krankhafte Zustände gerichtet sein, es können aber auch mehrere, gegebenenfalls ursächlich miteinander verbundene Anomalien zu Mustern, d.h. Syndromen, zusammengefaßt sein, die erfindungsgemäß behandelt werden können.

Zu den erfindungsgemäß behandelbaren Störungen gehören vor allem psychiatrische und neurologische Störungen. Hierzu zählen insbesondere organische Störungen, symptomatische Störungen eingeschlossen, wie Psychosen vom akuten exogenen Reaktionstyp oder Begleit-Psychosen organischer bzw. exogener Ursache, z.B. bei Stoffwechselstörungen, Infektionen und Endokrinopathien; endogene Psychosen, wie Schizophrenie sowie schizotype und wahnhafte Störungen; affektive Störungen, wie Depressionen, Manie bzw. manisch-depressive Zustände; sowie Mischformen der zuvor geschilderten Störungen; neurotische und somatoforme Störungen sowie Störungen bei Belastung; dissoziative Störungen, z.B. Bewußtseinsausfälle, -eintrübungen und -Spaltungen und Persönlichkeitsstörungen; Störungen von Aufmerksamkeit und Wach/Schlafverhalten, wie Verhaltensstörungen und emotionale Störungen, deren Beginn in der Kindheit und Jugend liegt, z.B. Hyperaktivität bei Kindern, intellektuelle Defizite, insbesondere Aufmerksamkeitsstörungen (attention deficit disorders), Gedächtnis- und kognitive Störungen, z.B. Lem- und Gedächtnisschwäche (impaired cognitive function), Demenz, Narkolepsie und Schlafstörungen, z.B. restless legs syndrome; Entwicklungsstörungen; Angstzustände; Delirium; Störungen des Sexuallebens, z.B. Impotenz des Mannes; Eßstörungen, z.B. Anorexie oder Bulimie; Sucht; und weitere nicht näher bezeichnete psychiatrische Störungen.

Zu den erfindungsgemäß behandelbaren Störungen gehören auch Parkinson und Epilepsie und insbesondere die damit in Zusammenhang stehenden affektiven Störungen.

Zu Suchterkrankungen gehören die durch den Missbrauch von psychotropen Substanzen, wie Arzneimittel oder Drogen, verursachte psychische Störungen und Verhaltensstörungen sowie andere Suchterkrankungen, wie beispielsweise die Spielsucht (Impulse Control Disorders not elsewhere classified). Suchterzeugende Substanzen sind beispielsweise: Opioide (z. B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartigen Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Im Hinblick auf die Behandlung von Suchterkrankungen sind unter den erfindungsgemäßen Verbindungen der Formel I solche besonders bevorzugt, die selbst keine psychotrope Wirkung besitzen. Dies kann im Test auch an Ratten beobachtet werden, die nach Verabreichung erfindungsgemäß brauchbarer Verbindungen die Selbstverabreichung von psychotropen Substanzen, beispielsweise Kokain, drosseln.

Gemäß einem weiteren Aspekt der vorliegenden Erfindung sind die erfindungsgemäßen Verbindungen zur Behandlung von Störungen geeignet, deren Ursachen zumindest zum Teil auf eine anomale Aktivität von Dopamin-D₃-Rezeptoren zurückzuführen sind.

Gemäß einem anderen Aspekt der vorliegenden Erfindung richtet sich die Behandlung vor allem auf diejenigen Störungen, die sich über eine Bindung von vorzugsweise exogen zugesetzten Bindungspartnern (Liganden) an Dopamin-D₃-Rezeptoren im Sinne einer zweckmäßigen medizinischen Behandlung beeinflussen lassen.

Die mit den erfindungsgemäßen Verbindungen behandelbaren Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäßen Verbindungen lassen sich eine Vielzahl von Anzeichen, Symptomen und/oder Fehlfunktionen behandeln, die mit den Störungen des zentralen Nervensystems und insbesondere den vorstehend genannten Zuständen zusammenhängen. Hierzu gehören beispielsweise ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Störungen der Merk- und Kombinationsfähigkeit, Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontanität und Entschlußkraft, verarmte Assoziationsfähigkeit, Angst, nervöse Unruhe, Stottern, soziale Phobie, Panikstörungen, Entzugssyndrome bei Abhängigkeit, maniforme Syndrome, Erregungs- und Verwirrtheitszustände, Dysphorie, dyskinetische Syndrome und Tic-Störungen, z.B. Chorea-Huntington, Gilles-de-la-Tourette-Syndrom, Schwindelsyndrome, z.B. peripherer Lage-, Dreh- und Schwankschwindel, Melancholie, Hysterie, Hypochondrie und ähnliches.

Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Vorzugsweise eignen sich die erfindungsgemäßen Verbindungen zur Behandlung von Erkrankungen des zentralen Nervensystems, insbesondere zur Behandlung von affektiven Störungen; neurotischen, Belastungs- und somatoformen Störungen und Psychosen und speziell zur Behandlung der Schizophrenie und der Depression. Aufgrund ihrer hohen Selektivität bezüglich des D₃-Rezeptors sind die erfindungsgemäßen Triazolverbindungen auch zur Behandlung von Nierenfunktionsstörungen, insbesondere von Nierenfunktionsstörungen, die durch Diabetes-Mellitus hervorgerufen wird (siehe WO 00/67847).

Die erfindungsgemäße Verwendung der beschriebenen Verbindungen beinhaltet im Rahmen der Behandlung ein Verfahren. Dabei wird dem zu behandelnden Individuum, vor zugsweise einem Säuger, insbesondere einem Menschen, Nutz- oder Haustier, eine wirksame Menge eines oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabfolgung gegebenenfalls zusammen oder im Wechsel mit anderen Wirkstoffen oder wirkstoff haltigen Präparaten, so daß einem zu behandelnden Individuum eine Tagesdosis von vorzugsweise etwa 1 bis 1000 mg/kg Körpergewicht bei oraler Gabe bzw. von etwa 0,1 bis 100 mg/kg Körpergewicht bei parenteraler Gabe zugeführt wird.

Die Erfindung betrifft auch die Herstellung pharmazeutischer Mittel zur Behandlung eines Individuums, vorzugsweise eines Säugers, insbesondere eines Menschen, Nutz- oder Haustieres. So werden die Liganden gewöhnlich in Form von pharmazeutischen Zusammensetzungen verabreicht, die einen pharmazeutisch verträglichen Exzipienten mit wenigstens einem erfindungsgemäßen Liganden und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Inhibitoren verwendet werden. Ferner können auch Liposomen oder Mikrosphären zur Anwendung kommen.

Bei der Herstellung der Zusammensetzungen werden erfindungsgemäße Inhibitoren gewöhnlich mit einem Exzipienten vermischt oder verdünnt Exzipienten können feste, halbfeste oder flüssige Materialien sein, die als Vehikel, Träger oder Medium für den Wirkstoff dienen.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner, Geruchsmaskierungsmittel; Geschmackskorrigentien; Harze; Hydrokolloide; Lösemittel; Lösungsvermittler; Neutralisierungsmittel; Permeationsbeschleuniger, Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher, Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die nachfolgenden Beispiele dienen zur Erläuterung der Erfindung ohne sie zu begrenzen.

Herstellungsbeispiele

### Beispiel I: 2-{3-[(4-Methyl-5-phenyl-4H-1,2,4-triazol-3-yl)thio]propyl}-7-propionyl-1,2,3,4-tetrahydroisochinolin (Hydrochlorid) - Verbindung la'-25 als Hydrochlorid

### 1.1 2-(Trifluoroacetyl)-1,2,3,4-tetrahydroisochinolin

Trifluoressigsäureanhydrid (2,13 mol, 452,0 g) wurde in Dichlormethan (452 ml) bei 10-15°C vorgelegt. Hierzu gab man bei dieser Temperatur eine Lösung von Tetrahydroisochinolin (1,94 mol, 268,3 g) in Dichlormethan (90 ml). Man rührte die Reaktionsmischung Ober Nacht bei Raumtemperatur nach und hydrolysierte anschließend mit Eiswasser (813 g). Nach 1 h Rühren wurden die Phasen getrennt. Die organische Phase wurde nacheinander mit Wasser (813 ml), mit halbkonzentrierter NaHCO₃-Lösung (550 ml) und erneut mit Wasser (500 ml) gewaschen. Anschließend engte man unter vermindertem Druck ein, wobei man 446 g Rohprodukt erhielt, das in der folgenden Umsetzung eingesetzt wurde.

### 1.2 1-[2-(Trifluoroacetyl)-1,2,3,4-tetrahydroisochinolin-7-yl]propan-1-on

Aluminiumtrichlorid (0,78 mol, 103,7 g) wurde in Dichlormethan (93 ml) bei 10-15°C suspendiert. Anschließend gab man bei dieser Temperatur unter Kühlung nacheinander das Trifluoracetyltetrahydrosochinolin aus Schritt 1.1 (2,13 mol, 452,0 g) und Propionsäurechlorid (0,47 mol, 43,2 g) zu. Anschließend erhitzte man zum Rückfluss und behielt die Temperatur 5 h bei. Danach kühlte man auf 5 -10°C ab und verdünnte mit 70 ml Dichlormethan. in einem 2 Kolben vorbereitet. Die Reaktionslösung wurde anschließend zügig unter Eisbadkühlung in eine Mischung aus 1000 g Eis und 500 ml Methyl-tert.-butylether eingebracht. Nach 30 min trennte man die Phasen und wusch die organische Phase nacheinander mit 500 ml Wasser, mit 500 ml halbkonzentrierter NaHCO₃-LÖsung und erneut mit 300 ml Wasser. Die organische Phase wurde anschliessend unter vermindertem Druck eingeengt; wobei man 89,9 g einer Mischung der Titelverbindung mit seinem 6-Isomer erhielt (Isomerenverhältnis 7-Isomer : 6-Isomer: etwa 75:25 (mittels ¹³C-NMR)), die in der Folgenden Stufe eingesetzt wurde.

### 1.3 7-Propionyl-1,2,3,4- tetrahydroisochinolin (Hydrochlorid)

Das Produkt aus Schritt 1.2 (0,39 mol, 111,0 g) löste man in n-Propanol (744 ml) und gab hierzu Salzsäure (32%ig, 3,5 mol, 400 g). Anschließend erhitzte man 5 h zum Rückfluß. Danach gab man weitere 300 ml n-Propanol zu und destillierte Wasser im Azeotrop mit n-Propanol ab. Insgesamt wurden 890 ml Destillat abdestilliert. Hierbei das Hydrochlorid des Propionylisochinolins aus; es wurden nochmals 1500 ml n-Propanol zugegeben und wieder abdestilliert. Anschließend wurden 1200 ml Methyl-tert.-butylether zugegeben, auf 5°C abgekühlt und 30 min gerührt. Der erhaltene Feststoff wurde abfiltriert und bei 40-50°C im Vakuum getrocknet. Man erhielt auf diese Weise 82,9 g, einer Mischung von 6- und 7-Propionyl-1,2,3,4- tetrahydroisochinolin als Hydrochlorid mit einem Isomerenverhältnis von 7-Isomer:6-Isomer von etwa 80:20 (bestimmt mittels ¹³C-NMR)).

### 1.4 1-[2-(3-Chloropropyl)-1,2,3,4-tetrahydroisochinolin-7-yl]propan-1-on

Das Isomerengemisch aus Schritt 1.3 (0,27 mol, 63,4 g) wurde in 634 ml Dichlor methan suspendiert. Zur Freisetzung der Base aus dem Hydrochlorid gab man 1,05 eq. Natronlauge und 317 ml Wasser zu und trennte die Phasen. Nach Phasentrennung und Extraktion der Wasserphase mit wenig Dichlormethan wurden die vereinigten organischen Phasen einmal mit 50 ml Wasser gewaschen, filtriert und mehrfach am Rotationsverdampfer eingeengt. Der Rückstand wurde in Dimethylformamid gelöst und die Lösung wurde nacheinander mit 1-Brom-3-chlorpropan (0,68 mol, 107,4 g) und Triethylamin (0,55 mol, 55,3 g) versetzt. Man rührte 16 h bei Raumtemperatur nach, gab zu der entstandenen Suspension 760 ml Wasser und extrahierte 2 mal mit etwa 300 ml Dichlormethan. Die vereinigten organischen Phasen wurden einmal mit 100 ml Wasser gewaschen, filtriert und unter vermindertem Druck eingeengt. Der Rückstand wurde in 61 ml Methanol und 410 ml Methyl-tert.-butylether gelöst. Dazu tropft man 1,2 Äquivalente Chlorwasserstoff in Isopropanol. Man kochte kurz auf und kühlte dann langsam auf Raumtemperatur ab. Man filtrierte den Niederschlag abfiltriert saugte Luft durch bis er gut war. Man erhielt auf diese Weise 87 g Rohprodukt, das zweimal aus Methanol umkristallisiert wurde. Auf diese Weise erhielt man 26.7g der Titelverbindung (7-Isomer : 6 Isomer: 97:3 (via ¹³C-NMR)).

### 1.5 Kalium-4-methyl-5-phenyl-4H-1,2,4-triazol-3-thiolat

Methylisothiocyanat (0.75mol, 54.5g) und Kaliumcarbonat (0.73mol, 101,5g) wurden in Ethanol (1.8 L) vorgelegt. Hierzu gab man bei Raumtemperatur Benzoesäurehydrazid (0.73mol, 100.0g). Die gelbliche Suspension wurde 3 h zum Rückfluss erhitzt, abgesaugt und der Filter wurde mit Methanol nahgewaschen. Das Filtrat wurde eingeengt und mit Dichlormethan/Methanol (1 Vol %) ausgerührt. Auf diese Weise erhielt man 89.4 g der Titelverbindung als hellen Feststoff. Man löste den Filterkuchen in Wasser (400ml), und extrahierte mit Essigester und Dichlormethan. Die organische Phase wurde getrocknet, filtriert und eingeengt. Auf diese Weise erhielt man weiter 44.4g der Titelverbindung als hellen Feststoff. Ausbeute: 133.8g.

### 1.5 2-{3-[(4-Methyl-5-phenyl-4H-1,2,4-triazol-3-yl)thio]propyl}-7-propionyl-1,2,3,4-tetrahydroisochinolin (Hydrochlorid)

1.00 g (3.76mmol) Kalium-4-methyl-5-phenyl-4H-1,2,4-triazol-3-thiolat aus Schritt 1.4 und 0.90g 1-[2-(3-Chloropropyl)-1,2,3,4-tetrahydroisochinolin-7-yl]propan-1-on (3.92mmol) aus Schritt 1.4 wurden mit Triethylamin (5.77mmol, 0.58g) in Dimethylformamid (30ml) bei Raumtemperatur über Nacht gerührt. Zur Aufarbeitung versetzte man mit einer Kochsalzlösung, extrahierte mit Essigester, trocknete die vereinigten organischen Phasen und entfernte das Lösungsmittel. Man erhielt so 1.5g eines dunkelbraunen Öls, das die Titelverbindung als freie Base enthält. Hieraus wurde das Hydrochlorid durch Fällung aus Isopropanot-HCI gewonnen. Man erhielt so 0.7g der Titelverbindung als beigefarbenen Feststoff mit einem Schmelzpunkt von 181-184°C.

EI-MS [M⁺] = 420;
*¹H-NMR (270 MHz, DMSO) δppm: 7.94-7.52 (m 7H), 7.41 (d, 1H), 4.79-4.52 (m br., 1H), 4.47-4.25 (m br., 1H), 3.71 (m br., 1H), 3.63 (s, 3H), 3.49-3.21 (m, 6H), 3.12 (m br., 1 H), 3.01 (m sym., 2H), 2.27 (quint., 2H), 1.07 (t, 3H).*

### Beispiel 2: 2-(3-{[4-Methyl-5-(1-methyl-1H-pyrrol-2-yl)-4H-1,2,4-triazol-3-yl]thio})propyl)-7-propionyl-1,2,3,4-tetrahydroisochinolin (Hydrochlorid) - Verbindung la'-15 als Hydrochlorid

20.38g (0,1 mol) Methyl-5-(1-methyl-1H-pyrrol-2-yl)-4H-1,2,4-triazol-3-thio (Rappoport et al. J. Org. Chem 37 (1972) 3618) wurde zusammen mit Lithiumhydroxid (0.36mol, 8.65g) in Dimethylformamid (130ml) vorgelegt. Hierzu gab man 26.00g (86 mmol) der Verbindung aus Beispiel 1.4 als Feststoff. Die Reaktionsmischung wurde zur Erzielung eines möglichst vollständigen Umsatzes etwa 2,5 -3 Tage bei Raumtemperatur nachgerührt. Zur Aufarbeitung wurde die Reaktionsmischung mit 390 ml Wasser versetzt und zweimal mit 130 ml Methyl-tert.-butylether/Ethylacetat (1:1 vol/vol) extrahiert. Die vereinigten organischen Phasen wurden mit 100 ml verdünnter Kochsalz-Lösung gewaschen. Die organische Phase wurde mit Aktivkohle verrührt und danach filtriert. Durch Einengen des Filtrats 39,8g Rohprodukt. Das Rohprodukt löste man in 138 ml Methanol, gab hierzu bei Raumtemperatur 3 Äquivalente Chlorwasserstoff, bezogen auf die eingesetzte Verbindung aus Beispiel 1.4, als Lösung in Isopropanol, rührte die entstandene Suspension über Nacht nach, kühlte dann auf 5°C abgekühlt und filtriert den Feststoff ab. Der Feststoff wurde bei 40-50°C im Vakuum getrocknet, wobei man 24,7 g der Titelverbindung als Hydrochlorid erhielt, das aus Methanol (59 ml) umkristallisiert wurde. Das Produkt wurde erneut bei 40-50°C im Vakuum getrocknet, wobei man 16,4 g der Titelverbindung als Hydrochlorid mit einem Schmelzpunkt von 181-184°C erhielt;
EI-MS [M⁺] = 423; *¹H-NMR (270 MHz, DMSO) δ ppm: 7.91-7.78 (m 2H), 7.39 (d, 1H), 7.08 (s, 1H), 6.62 (m, 1H), 6.22 (m, 1H), 4.77-4.50 (m br., 1 H), 3. 78 (s, 3H), 3.71 (m br., 1H), 3.62 (s, 3H), 3.32 (m, 6H), 3.11 (m br., 1H), 3.01 (m sym., 2H), 2.27 (quint., 2H), 1.07 (t, 3H).*

### Beispiel 3: 7-Benzoyl-2-{3-[(4-methyl-5-phenyl-4H-1,2,4-triazol-3-yl)thio]propyl}-1,2,3,4-tetrahydroisochinoline (Hydrochlorid) - Verbindung la'-19 als Hydrochlorid

### 3.1 N-(2-Phenylethyl)acetamid

Phenetylamin (206.30mmol, 25.00g) wurde in Toluol (300ml) vorgelegt. Acetylchlorid (252.23mmol, 19.80g) wurde zugetropft und der Ansatz über Nacht bei Raumtemperatur nachgerührt. Die Reaktionsmischung wurde auf Wasser gegeben und die wässrige Mischung wurde mit Essigester extrahiert. Die organische Phase wurde mit gesättigter wässriger Kochsalz -Lösung gewaschen, getrocknet und eingeengt Man erhielt 32.7g der Titelverbindung als braunes Öl, das an Kieselgel (Laufmittel Dichlormethan mit 1% Methanol) chromatographiert und in Hexan ausgerührt wurde. Hierbei erhielt man 27.6g der Titelverbindung mit einem Schmelzpunkt von 50-53°C.

### 3.2 N-[2-(4-Benzoylphenyl)ethyl]acetamid

*N*-(2-Phenylethyl)acetamid (49.01mmol, 8.00g) wurde in Nitrobenzol (40ml) vorgelegt. Hierzu gab man Benzoylchlorid (54.07mmol, 7.60g) und trug dann bei Raumtemperatur Aluminiumtrichlorid (73.52mmol, 9.80g) portionsweise ein. Anschliessend erhitzte man 8 h auf 50°C. Die Reaktionsmischung wurde auf eine Mischung aus Eis und konzentrierter Salzsäure gegeben und Nitrobenzol wurde durch Wasserdampfdestillation entfernt. Der wässrige Rückstand wurde mit Essigester extrahiert. Nach Einengen der organischen Phase wurde der Rückstand an Kieselgel (Laufmittel Dichlormethan/Methanol (3 Vol.%)) chromatographiert, wobei man 4.5g der Titelverbindung als orangenes Öl erhielt.

### 3.3 (2-Acetyl-1,2,3,4-tetrahydroisochinolin-7-yl)(phenyl)methanon

*N*-[2-(4-Benzoylphenyl)ethyl]acetamid (16.46mmol, 4.40g) wurde in Eisessig (20ml) vorgelegt. Hierzu gab man langsam bei 30°C konzentrierte Schwefelsäure (25ml) und anschließend Formalin (49.29mmol, 4.00g). Die Mischung wurde 2 Tage nachgerührt, auf Eiswasser gegeben und die wässrige Phase wurde mit Essigester extrahiert. Die organische Phase wurde mit wässriger Natriumcarbonat-Lösung gewaschen, getrocknet und eingeengt. Der.Rückstand wurde mit Diethylether verrührt und der dabei anfallende Feststoff wurde abgesaugt. Man erhielt 1.6g der Titelverbindung als beigefarbenen Feststoff mit einem Schmelzpunkt von 110-113°C.

### 3.4 Phenyl(1,2,3,4-tetrahydroisochinolin-7-yl)methanon

(2-Acetyl-1,2,3,4-tetrahydroisochinolin-7-yl)(phenyl)methanon (11.10 mmol, 3.10g) wurde in Salzsäure (7M, 250ml) 6h zum Rückfluss erhitzt. Man engte die erhaltene Mischung ein, stellte mit Natromlauge alkalisch und extrahierte mit Essigester. Nach Trocknen der organische Phase und einengen erhielt man 1.7g der Titelverbindung als braunes Öl.

### 3.5 [2-(3-Chloropropyl)-1,2,3,4-tetrahydroisochinolin-7-yl](phenyl)methanon

Analog zu der Vorschrift für Schritt 1.4 aus Beispiel 1 erhielt man ausgehend von Phenyl(1,2,3,4-tetrahydroisochinolin-7-yl)methanon (7.16mmol, 1.70g) 1.3g der Titelverbindung als orangenes Öl.

### 3.6 7-Benzoyl-2-{3-[(4-methyl-5-phenyl-4H-1,2,4-triazol-3-yl)thio]propyl}-1,2,3,4-tetrahydroisochinolin (Hydrochlorid)

Analog zu der Vorschrift für Beispiel 2 erhielt man durch Umsetzung der in Stufe 3.5 erhaltenen Verbindung (2.23mmol, 0.70g) mit dem Triazol aus Schritt 1.5 (2.35mmol, 0.45g) 0.50g der Titelverbindung als beigefarbenen Feststoff mit einem Schmelzpunkt von 146-152°C.

*¹H-NMR (270 MHz, DMSO) δ ppm: 7.81-7.50 (m 12H), 7.44 (d, 1 H), 4.79-4.53 (m br., 1H), 4.48-4.26 (m br., 1H), 3.66 (s, 3H), 3.37 (m, 6H), 3.16 (m br., 1H), 2.28 (quint., 2H).*

### Beispiel 4: 7-Benzoyl-2-{4-[(4-methyl-5-phenyl-4H-1,2,4-triazol-3-yl)thio]butyl}-1,2,3,4-tetrahydroisochinoline (Hydrochlorid) - Verbindung la'-77 als Hydrochlorid

### 4.1 3-[(4,4-Dimethoxybutyl)thio]-4-methyl-5-phenyl-4H-1,2,4-triazol

Das Triazol aus Schritt 1.5 (43.60mmol, 10.00g) und 4-Chlor-1,1-dimethoxybutan (44.55mmol, 6.80g) wurden 10h bei 80°C gerührt. Die Reaktionsmischung wurde auf Wasser gegeben und mit Essigester extrahiert. Die organische Phase wurde mit wässriger Kochsalz-Lösung gewaschen, getrocknet und eingeengt. Der Rückstand wurde chromatographisch (Laufmittel: Ethylacetat) gereinigt. Man erhielt 6.3 g der Titelverbindung als farbloses Öl.

### 4.2 4-[(4-Methyl-5-phenyl-4H-1,2,4-triazol-3-yl)thio]butanal

Zu einer Lösung von 3-[(4,4-Dimethoxybutyl)thio]-4-methyl-5-phenyl-4H 1,2,4-triazol (20.49mmol, 6.30g) in Ethanol (50ml) gab man konzentrierte Schwefelsäure bis zur Trübe. Die Mischung wurde 2h bei 40°C gerührt, anschliessend mit Natriumcarbonat alkalisch gestellt und danach mit Essigester extrahiert. Die organische Phase wurde getrocknet und eingeengt, wobei man 4.8g der Titelverbindung farbloses Öl erhielt.

### 4.3 7-Benzoyl-2-{4-[(4-methyl-5-phenyl-4H-1,2,4-triazol-3-yl)thio]butyl}-1,2,3,4-tetrahydroisochinoline (Hydrochlorid)

4-[(4-Methyl-5-phenyl-4*H*-1,2,4-triazol-3-yl)thio]butanal (3.83mmol, 1.00g), das Hydrochlorid aus Schritt 3.3 (3.65mmol, 1.00g) und Eisessig (14.99mmol, 0.90g) wurden in Methanol vorgelegt. Hierzu gab man bei Raumtemperatur portionsweise NaCNBH₃ (4.77mmol, 0.30g). Die Mischung wurde bei Raumtemperatur über Nacht nachgerührt und anschliessend auf Wasser gegeben. Man stellte mit Na₂CO₃ alkalisch und extrahierte mit Ethylacetat. Die organische Phase wurde mit einer wässrigen Kochsalz-Lösung gewaschen, getrocknet und eingeengt, wobei man 1.7g eines orangefarbenen Öls erhielt, das chromatographisch an Kieselgel (Laufmittel: Dichlormethan/Methanol (1.5-3.5 Vol.-%)) gereinigt wurde. Man erhielt 1.1g der freien Base der Titelverbindung als gelbes Öl, das mit Chlorwasserstoff in Isopropanol in das Hydrochlorid überführt wurde. Man erhielt 0.9g Hydrochlorid als weissen Feststoff mit einem Schmelzpunkt von 171 °C.

*¹H-NMR (360 MHz, DMSO) δ ppm: 7.77-7.52 (m 12H), 7.42 (d, 1H), 4.63 (d br., 1H), 4.41-4.23 (m br., 1H), 3.69 (s br., 1H), 3.61 (s, 3H), 3.42-3.04 (m, 7H), 1.94 (quint., 2H), 1.80 (quint., 2H).*

### Beispiel 5: 7-Benzoyl-2-(3-{[4-methyl-5-(1-methyl-1H-pyrrol-2-yl)-4H-1,2,4-triazol-3-yl]thio}propyl)-1,2,3,4-tetrahydroisochinolin (Hydrochlorid) - Verbindung la'-12 als Hydrochlorid

Analog zu der Vorschrift für Beispiel 2 erhielt man durch Umsetzung der in Schritt 3.5 hergestellten Verbindung (1.59mmol, 0.50g) mit Methyl-5-(1-methyl-1H-pyrrol-2-yl)-4H-1,2,4-triazol-3-thiol (1.54mmol, 0.30g) 0.18g der Titelverbindung als weissen Feststoff mit einem Schmelzpunkt von 138°C.
*¹H-NMR (360 MHz, DMSO) δ ppm: 7.75-7.50 (m 7H), 7.42 (d, 1H), 7.03 (s, 1H), 6.55 (m, 1H), 6.17 (m, 1 H), 4. 67 (s br., 1H), 4.37 (s br., 1H), 3.76 (s br., 3+1H), 3. 60 (s, 3H), 3.30 (m, 6H), 3.15 (s br., 1H), 2.27 (quint., 2H).*

### Beispiel 6: 7-Benzoyl-2-(3-{[4-methyl-5-(1H-pyrrol-2-yl)-4H-1,2,4-triazol-3-yl]thio}propyl)-1,2,3,4-tetrahydroisochinolin (Hydrochlorid) - Verbindung la'-64 als Hydrochlorid

Analog zu der für Beispiel 2 angegebenen Vorschrift erhielt man durch Umsetzung von 4-Methyl-5-(1*H*-pyrrol-2-yl)-4*H*-1,2,4-triazole-3-thiol (1.59mmol, 0.50g) mit der in Schritt 3.5 hergestellten Tetrahydroisochinolinverbindung (1.66mmol, 0.30g) 0.20g der Titelverbindung als weissen Feststoff mit einem Schmelzpunkt von 163°C;
*¹H-NMR (360 MHz, DMSO) δppm: 7 73-7. 52 (m 7H), 7.41 (d, 1 H); 7.05 (s, 1 H), 6.74 (m, 1H), 6.27 (m, 1H), 4.64 (s br., 1H), 4.36 (s br., 1H), 3. 71 (s, 3H), 3.41-3.21 (m, 5H), 3.15 (m br., 1H), 2.19 (quint., 2H).*

### Beispiel 7:7-Benzoyl-2-(3-{[5-(2-furyl)-4-methyl-4H-1,2,4-triazol-3-yl]thio}propyl)-1,2,3,4-tetrahydroisochinolin (Hydrochlorid) - Verbindung la'.3 als Hydrochlorid

Analog zu der für Beispiel 2 angegebenen Vorschrift erhielt man durch Umsetzung von der in Schritt 3.5 hergestellten Tetrahydroisochinolinverbindung (1.27mmol, 0.40g) und 5-(2-Furyl)-4-methyl-4*H*-1,2,4-triazol-3-thiol (1.38mmol, 0.25g; Herstellung analog zu Schritt 2.3) 0.10g der Titelverbindung als gelblichen Feststoff mit einem Schmelzpunkt von 180-185°C;
*¹H-NMR (360 MHz, DMSO) δ ppm: 7.96 (s, 1H), 7.76-7.54 (m 7H), 7.43 (m, 1 H), 7.09 (m, 1H), 6.73 (m, 1H), 4.69 (d br., 1H), 4.44-4. 27 (m br., 1H), 3.72 (s, 3H), 3.35 (m br., 2H), 3. 28 (m, 6H), 3.16 (m br., 1H), 2. 20 (quint., 2H).*

### Beispiel 8: 7-Benzoyl-2-{3-[(4-methyl-5-thien-3-yl-4H-1,2,4-triazol-3-yl)thio]propyl}-1,2,3,4-tetrahydroisochinolin (Hydrochlorid) - Verbindung Ia'-18 als Hydrochlorid

Analog zu der für Beispiel 2 angegebenen Vorschrift erhielt man durch Umsetzung von der in Schritt 3.5 hergestellten Tetrahydroisochinolinverbindung (1.27mmol, 0.40g) und Natrium 4-methyl-5-thien-2-yl-4*H*-1,2,4-triazole-3-thiolat (1.37mmol, 0.30g; Herstellung analog zu Schritt 1.5) 0.10g der Titelverbindung als gelblichen Feststoff mit einem Schmelzpunkt von 140°C;
EI-MS [M⁺] = 474; *¹H-NMR (360 MHz, DMSO) δppm: 11.10 (s br., 1H), 8.15 (s, 1H), 7. 84-7. 55 (m 9H), 7.45 (d, 1H), 4. 68 (s br., 1H), 4.39 (s br., 1H), 3. 71 (s, 3H), 3.50-3.24 (m, 6H), 3.19 (m br., 1H), 2.25 (quint., 2H).*

### Beispiel 9: 7-Benzoyl-2-{3-[(4-methyl-5-pyridin-3-yl-4H-1,2,4-triazol-3-yl)thio]propyl}-1,2,3,4-tetrahydroisochinolin (Hydrochlorid) - Verbindung la'-64 als Hydrochlorid

Analog zu der für Beispiel 2 angegebenen Vorschrift erhielt man durch Umsetzung von der in Schritt 3.5 hergestellten Tetrahydroisochinolinverbindung (1.27mmol, 0.40g) und 3-(5-Mercapto-4-methyl-4*H*-1,2,4-triazol-3-yl)pyridinium chlorid (1.31mmol, 0.30g; Herstellung analog zu Schritt 2.3) 0.17g der Titelverbindung als weissen Feststoff mit einem Schmelzpunkt von 148°C; *¹H-NMR (360 MHz, DMSO) 8 ppm: 9.00 (s, 1H), 8.83 (m, 1H), 8.33 (m, 1H), 7. 78-7. 53 (m 8H), 7.44 (d, 1H), 4. 75-4. 62 (m br., 1H), 4.43-4.29 (m br., 1H), 3.73 (m br., 1H), 3.68 (s, 3H), 3.43 (m, 6H), 3.19-3.08 (m, 1H), 2.25 (quint., 2H).*

### Beispiel 10: 7-Benzoyl-2-{3-[(4-methyl-5-pyridin-4-yl-4H-1,2,4-triazol-3-yl)thio]propyl}-1,2,3,4-tetrahydroisochinolin (Hydrochlorid) - Verbindung Ia'-1 als Hydrochlorid

Analog zu der für Beispiel 2 angegebenen Vorschrift erhielt man durch Umsetzung von der in Schritt 3.5 hergestellten Tetrahydroisochinolinverbindung (1.27mmol, 0.40g) und 4-(5-Mercapto'-4-methyl-4*H*-1,2,4-triazol-3-yl)pyridinium chlorid (1.31 mmol, 0.30g; Herstellung analog zu Schritt 2.3) 0.30g der Titelverbindung als gelblichen Feststoff mit einem Schmelzpunkt von 140-146°C;
*¹H-NMR (360 MHz, DMSO) δ ppm: 11.40 (s br., 1H), 8.95 (m, 2H), 8.10 (m 2H), 7.79-7.56 (m, 7H), 7.45 (d, 1H), 4.81-4.13 (m, 2H), 3.79 (s, 3H), 3. 79 3. 69 (m, 1H), 3.48-3.29 (m, 6H), 3.22-3.10 (m, 1H), 2.35 (quint, 2H).*

### Beispiel 11: 7-(3-Fluorobenzoyl)-2-{3-[(4-methyl-5-pyrazin-2-yl-4H-1,2,4-triazol-3-yl)thio]-propyl}-1,2,3,4-tetrahydroisochinolin (Hydrochlorid) - Verbindung la'-58 als Hydrochlorid

### 11.1 N-{2-[4-(3-Fluorobenzoyl)phenyl]ethyl}acetamid

Analog zu der für Schritt 3.2 angegebenen Vorschrift erhielt man durch Umsetzung der in Schritt 3.1 hergestellten Verbindung (61.27mmol, 10.00g) mit 3-Fluorbenzoylchlorid (67.48mmol, 10.70g) 8.60g *N*-{2-[4-(3-Fluorobenzoyl)phenyl]ethyl}acetamid als beigefarbener Feststoff mit einem Schmelzpunkt von 78°C.

### 11.2 (2-Acetyl-1,2,3,4-tetrahydroisochinolin-7-yl)(3-fluorophenyl)methanon

Analog zu der für Schritt 3.3 angegebenen Vorschrift stellte man aus *N*-{2-[4-(3-Fluorobenzoyl)phenyl]ethyl}acetamid (5.60g, 19.63mmol) 2.60g der Titelverbindung hergestellt; beigener Feststoff, Schmelzpunkt 136-139°C.

### 11.3 7-(3-Fluorobenzoyl)-1,2,3,4-tetrahydroisochinolin (Hydrochlorid)

Analog zu der für Schritt 3.3 angegebenen Vorschrift stellte man aus der in Schritt. 11.2 erhaltenen Verbindung (8.74mmol, 2.60g) 2.20g der Titelverbindung her; beigener Feststoff, Schmelzpunkt 219-223°C.

### 11.4 [2-(3-Chloropropyl)-1,2,3,4-tetrahydrolsochinolin-7-yl](3-fluorophenyl)methanon

Analog zu der für Schritt 3.4 angegebenen Vorschrift stellte man aus der in Schritt 11.3 erhaltenen Verbindung (7.54mmol, 2.20g) 2.20g der Titelverbindung her; orangenes Öl.

### 11.5 7-(3-Fluorobenzoyl)-2-{3-[(4-methyl-5-pyrazin-2-yl-4H-1,2,4-triazol-3-yl)thio]propyl}-1,2,3,4-tetrahydroisochinoline (Hydrochlorid)

Analog zu der für Beispiel 2 angegebenen Vorschrift erhielt man durch Umsetzung von der in Schritt 11.4 hergestellten Tetrahydroisochinolinverbindung (1.27mmol, . 0.40g) und (1.51mmol, 0.50g) und 4-Methyl-5-pyrazin-2-yl-4*H*-1,2,4-triazole-3-thiol (1.55mmol, 0.30g) 0,20 g der Titelverbindung als weissen Feststoff der aus IsopropanollWasser 98:2 umkristallisiert wurde; EI-MS [M⁺] = 488

### Beispiel 12: 7-(3-Fluorobenzoyl)-2-{3-[(4-methyl-5-pyridin-3-yl-4H-1,2,4-triazol-3-yl)thio]-propyl}-1,2,3,4-tetrahydroisochinoline (Hydrochlorid) - Verbindung Ia'-63 als Hydrochlorid

Analog zu der für Beispiel 2 angegebenen Vorschrift erhielt man durch Umsetzung von der in Schritt 11.4 hergestellten Tetrahydroisochinolinverbindung (1.81 mmol, 0.60g) und 3-(5-Mercapto-4-methyl-4*H*-1,2,4-triazol-3-yl)pyridiniumchlorid (1.75mmol, 0.40g; Herstellung analog zu Schritt 2.3) 0.45g der Titelverbindung als beigefarbener Feststoff mit einem Schmelzpunkt von 133°C;
*¹H-NMR (360 MHz, DMSO) δ ppm: 11.54 (s br., 1H), 9.10 (s, 1H), 8.89 (m sym., 1H), 8.49 (m, 1H), 7.88 (m, 1H), 7.67-7.39 (m, 7H), 4.66 (m br., 1H), 4.41-4.31 (m, 1H), 3.70 (s, 3H), 3.46-3.27 (m, 6H), 3.12 (d br., 1H), 2.28 (quint., 2H).*

### Beispiel 13: 7-Acetyl-2-{3-[(4-methyl-5-thien-3-yl-4H-1,2,4-triazol-3-yl)thio] propyl}-1,2,3,4-tetrahydroisochinolin (Hydrochlorid) - Verbindung Ia'-13 als Hydrochlorid

### 13.1 1-[2-(Trifluoroacetyl)-1,2,3,4-tetrahydroisochinolin-7-yl]ethanon

2-(Trifluoroacetyl)-1,2,3,4-tetrahydroisochinolin (140 mmol, 32.00g) wurde in CS₂ (190m1) vorgelegt. Hierzu gab man bei Raumtemperatur portionsweise Aluminiumtrichlorid (837 mmol, 111.60g). Anschließend tropfte man Acetylchlorid (419.12mmol, 32.90g) so zu, dass leichter Rückfluss bestand und erhitzte eine weitere Stunde zum Rückfluss. Die Reaktionsmischung wurde auf Eiswasser gegeben und mit Dichlormethan extrahiert. Die organische Phase wurde getrocknet, abgesaugt und eingeengt und anschliessend mit Isopropanol ausgerührt. Man erhielt 22 g der Titelverbindung mit einem Schmelzpunkt von 80°C.

### 13.2 7-Acetyl-1,2,3,4-tetrahydroisochinolin (Hydrochlorid)

Analog zu der für Schritt 1.3 angegebenen Vorschrift stellte man aus der in Schritt 13.1 erhaltenen Verbindung (22.12mmol, 6.00g) 3.80g der Titelverbindung her (gelber Feststoff).

### 13.3 1-[2-(3-Chloropropyl)-1,2,3,4-tetrahydroisochinolin-7-yl]ethanon

Analog zu der für Schritt 1.4 angegebenen Vorschrift stellte man aus der in Schritt 13.2 erhaltenen Verbindung (17 mmol, 3.60g) 3.00g der Titelverbindung her (gelbes Öl).

### 13.4 7-Acetyl-2-{3-[(4-methyl-5-thien-3-yl-4H-1,2,4-triazol-3-yl)thio]propyl}-1,2,3,4-tetrahydroisochinolin (Hydrochlorid)

Analog zu der für Schritt 1.6 angegebenen Vorschrift erhielt man durch Umsetzung von der in Schritt 13.3 hergestellten Tetrahydroisochinolinverbindung (1.99mmol, 0.50g) mit Natrium 4-methyl-5-thien-2-yl-4*H*-1,2,4-triazol-3-thiolat (2.28mmol, 0.50g; Herstellung analog zu Schritt 1.5) 0.20g der Titelverbindung; EI-MS [M⁺] = 412.

### Beispiel 14: 7-(3-Fluorobenzoyl)-2-{3-[(4-methyl-5-phenyl-4H-1,2,4-triazol-3-yl)thio]propyl}-1,2,3,4-tetrahydroisochinoline (Hydrochlorid) - Verbindung Ia'-39 als Hydrochlorid

Analog zu der für Beispiel 2 angegebenen Vorschrift erhielt man durch Umsetzung von der in Schritt 11.4 hergestellten Tetrahydroisochinolinverbindung (1.51mmol, 0.50g) mit der Triazolverbindung aus Schritt 1.5 (1.51 mmol, 0.35g) 0.45g der Titelverbindung als beigefarbener Feststoff mit einem Schmelzpunkt von 147°C;
EI-MS [M⁺] = 486; *¹H-NMR (360 MHz, DMSO) δppm: 7.80-7.43 (m, 12H), 4.77-4.58 (m br., 1H), 4.45-4.27 (m br., 1H), 3.64 (s, 1H), 3.46-3.22 (m, 6H), 3.15 (m br., 1H), 2.24 (quint., 2H).*

### Beispiel 15: 7-(3-Fluorobenzoyl)-2-(3-{[4-methyl-5-(1-methyl-1H-pyrrol-2-yl)-4H-1,2,4-triazol-3-yl]thio}propyl)-1,2,3,4-tetrahydroisochinolin (Hydrochlorid) Verbindung la'-60 als Hydrochlorid

Analog zu der für Beispiel 2 angegebenen Vorschrift erhielt man durch Umsetzung von der in Schritt 11.4 hergestellten Tetrahydroisochinolinverbindung (1.81mmol, 0.60g) mit der Triazolverbindung aus Schritt 2.4 (1.81 mmol, 0.35g) 0.15g der Titelverbindung als beigefarbener Feststoff mit einem Schmelzpunkt von 139°C;
*¹H-NMR (400 MHz, DMSO) δppm: 11.78 (s br., 1H), 7.70-7.41 (m, 7H),* 7.12 *(s, 1H), 6. 67 (s, 1 H),* 6.23 *(m, 1H), 4.75-4.60 (m, 1H), 4.47-4.31 (m, 1H), 3.81 (s, 3H), 3.64 (s, 3H), 3.52-3.30 (m, 6H), 3.19-3.06 (m br., 1H), 2.31 (quint., 2H).*

### Beispiel16: 1-[2-(3-{[5-(1-Benzothien-2-yl)-4-methyl-4H-1,2,4-triazol-3-yl]thio}propyl)-1,2,3,4-tetrahydroisochinolin-7-yl]propan-1-on (Verbindung Ia'-57)

### 16.1 5-(1-Benzothien-2-yl)-4-methyl-4H-1,2,4-triazol-3-thiol

2-(1-Benzothien-2-ylcarbonyl)-N-methylhydrazincarbothioamid (15.30mmol, 4.06g) und 50 ml 2N Natronlauge wurden 8h zum Rückfluss erhitzt. Die Rekationsmischung wurde mit Wasser verdünnt und mit 3mal mit Ethylacetat extrahiert. Die wässrige Phase wurde mit 2N Salzsäure sauer gestellt und mit Dichlormethan extrahiert. Das nach Trocknen und Einengen der organischen Phase erhaltene Rohprodukt wurde chromatographisch an Kieselgel (Laufmittel: Cyclohexan/Ethylacetat 6:4) gereinigt, wobei man 2.2 g der Titelverbindung erhielt.

### 16.2 1-[2-(3-{[5-(1-Benzothien-2-yl)-4-methyl-4H-1,2,4-triazol-3-yl]thio}propyl)-1,2,3,4-tetrahydroisochinolin-7-yl]propan-1-on

Analog zu der für Beispiel 2 angegebenen Vorschrift erhielt man durch Umsetzung von der in Schritt 16.1 hergestellten Triazolverbindung (2.02 mmol, 0.50g) und der Isochinolinverbindung aus Schritt 1.4 (2.02mmol, 0.54g) 0.37g der Titelverbindung; EI-MS [M⁺] = 476.

### Beispiel 17: 2-(3-{[5-(1,3-Benzothiazol-2-yl)-4-methyl-4H-1,2,4-triazol-3-yl]thio}propyl)-7-propionyl-1,2,3,4-tetrahydroisochinoline (Hydrochlorid) - Verbindung Ia' - 70 als Hydrochlorid

Analog zu der für Beispiel 2 angegebenen Vorschrift erhielt man durch Umsetzung von 5-(1,3-Benzothiazol-2-yl)-4-methyl-4H-1,2,4-triazol-3-thiol (4.87mmol, 1.21g; Herstellung analog zu Schritt 16.1) und der Isochinolinverbindung aus Schritt 1.4 (4.87mmol, 1.30g) 0.47g der Titelverbindung; EI-MS [M⁺] = 477.

### Beispiel 18: 7-Acetyl-2-(3-{[4-methyl-5-(1-methyl-1H-pyrrol-2-yl)-4H-1,2,4-triazol-3-yl]thio}-propyl)-1,2,3,4-tetrahydroisochinolin (Hydrochlorid) - Verbindung la'-4 als Hydrochlorid

Analog zu der für Beispiel 2 angegebenen Vorschrift erhielt man durch Umsetzung von der in Schritt 13.3 hergestellten Tetrahydroisochinolinverbindung (2.78mmol, 0.70g) mit Methyl-5-(1-methyl-1H-pyrrol-2-yl)-4H-1,2,4-triazol-3-thiol (2.83mmol, 0.55g) 0.7g der Titelverbindung als beigefarbener Feststoff mit einem Schmelzpunkt von 152-158°C;
*¹H-NMR (270 MHz, DMSO) δ ppm: 11.20 (s br., 1H), Z91-Z78 (m, 2H), 7.40 (d, 1H), 7.07 (s, 1H), 6.60 (m, 1H), 6.22 (t, 1H), 4. 76-4. 54 (m br., 1H), 4.45-4.23 (m br., 1H), 3.60 (s, 3H), 3.45-3.19 (m, 6H), 3.12 (m br., 1H), 2.55 (s, 3H), 2.26 (quint., 2H).*

### Beispiel 19: 7-Acetyl-2-{3-[(4-methyl-5-pyridin-3-yl-4H-1,2,4-triazol-3-yl)thio]propyl}-1,2,3,4-tetrahydroisochinoline (Hydrochlorid) - Verbindung la'-39 als Hydrochlorid

Analog zu der für Beispiel 2 angegebenen Vorschrift erhielt man durch Umsetzung von der in Schritt 13.3 hergestellten Tetrahydroisochinolinverbindung (5.16mmol, 1.30g) mit 4-Methyl-5-pyridin-3-yl-4*H*-1,2,4-triazol-3-thiol Hydrochlorid (5.47mmol, 1.25g; Herstellung analog zu Schritt 2.3) 0.8g der Titelverbindung als hellen Feststoff; EI-MS [M⁺] = 407.

### Beispiel 20: 1-{2-[3-(4-Methyl-5-pyridin-2-yl-4H [1,2,4]triazol-3-ylsulfanyl)-propyl]-1,2,3,4-tetrahydroisochinolin-7-yl}-propan-1-on (Dihydrochlorid) - Verbindung, la'-71 als Dihydrochlorid

### 20.1 4-Methyl-5-pyridin-2-yl-4H-[1,2,4]triazol-3-thiol

15 g Pyridin-2-carbonsäure (121,8 mmol) wurden in 150 ml Dimethylformamid gelöst. Bei Raumtemperatur gab man langsam 29,6 g (181,8 mmol) Carbonyldiimidazol zu, wobei eine Gasentwicklung stattfand. Man liess 30 min. bei 100°C reagieren und gab dann 25,6 g (242,7 mmol) 4-Methyl-3-thiosemicarbazid zu und rührte weitere 2h bei 100°C nach. Die Reaktionsmischung wurde mit 300 ml Wasser versetzt. Nach Kühlung im Eisbad wurden die ausgefallenen Kristalle abgesaugt und mit wenig Wasser gewaschen. Das Kristallisat wurde in 390 ml 1M Natriumhydrogencarbonat-Lösung aufgenommen und 3h zum Rückfluss erhitzt. Nach Abkühlung neutralisierte man mit konzentrierter Salzsäure, filtrierte die weissen Kristalle ab, wusch mit wenig Wasser trocknete über Nacht bei 50°C, Man erhielt 18,5 g der Titelverbindung.

### 20.2 1-{2-[3-(4-Methyl-5-pyridin-2-yl-4H-[1,2,4]triazol-3-ylsulfanyl)-propyl]-1,2,3,4-tetrahydroisochinolin-7-yl}-propan-1-one (Dihydrochlorid)

1,08g (5.44mmol) 4-Methyl-5-pyridin-2-yl-4*H*-[1,2,4]triazole-3-thiol; 0,135g (5.44mmol) Lithiumhydroxid und 0,422g (2.82mmol) Natriumiodid legte man in 14 ml Dimethylformamid vor und erwärmte auf 70°C. Hierzu tropfte man innerhalb 2 h bei einer Temperatur von 70°C eine Lösung von 1,5g (5.44mmol) 1-[2-(3-Chloro-propyl)-1,2,3,4-tetrahydro-isochinolin-7-yl]-propan-1-on aus Schritt 1.4 in 6ml trockenem Dimethylformamid und rührte 30min. bei 90°C nach. Die Reaktionsmischung wurde im Vakuum eingeengt. Das hierbei erhaltene hellgelbe Öl gab man auf 20ml gesättigte Kochsalzlösung und 20ml Wasser und extrahierte anschliessend dreimal mit 30ml Ethylacetat. Die vereinigten organischen Phasen wurden über Magnesiumsulfat getrocknet, filtriert und einrotiert. Das so erhaltene Rohprodukt (2,8g) wurde mittels und einrotiert. Das so erhaltene Rohprodukt (2,8g) wurde mittels Flashchromatographie gereinigt. (Eluenten: 1. Dichlormethan; 2. Dichlormethan/Methanol 98:2; 3. Dichlormethan/Methanol 30: 1). Die hierbei erhaltene freie Base der Titelverbindung (1,1 g) wurde zur Herstellung des Hydrochloridsalzes in20ml Essigsäureethylester gelöst und die Lösung wurde mit 4N Chlorwasserstoff in Dioxan versetzt. Der ausgefallene weiße Niederschlag wurde abgesaugt, mit Ether gewaschen und getrocknet. Man erhielt 1.0g der Titelverbindung als weißen Feststoff; [M+H]+ = 424.

### Beispiel 21: 1-[2-(3-{[4-Methyl-5-(1H-pyrrol-2-yl)-4H-1,2,4-triazol-3-yl]thio}propyl)-2,3-dihydro-1H-isoindol-5-yl]propan-1-on Hydrochlorid

Analog zu Vorschrift 1.5 stellte man aus 4-Methyl-5-(1*H*-pyrrol-2-yl)-4*H*-1,2,4-triazole-3-thiol (1.43mmol, 0.26g) und 1-[2-(3-Chloropropyl)-2,3-dihydro-1*H*-isoindol-5-yl]propan-1-on (1.43mmol, 0.36g; Herstellung analog zu 1.4 aus Isoindolin nach WO 0125200) die Titelverbindung her. Ausbeute: 0.11g.

*¹H-NMR (500 MHz, CDCl₃) δ ppm: 7. 79 (d, 1H), 7.74 (s, 1H), 7.20 (d, 1H), 7.10 (s, 1H), 6.54 (s, 1H), 6.28 (m, 1H), 3.94 (s, 4H), 3.72 (s, 3H), 3.28 (t, 2H), 2.95 (q, 2H), 2.89 (t, 2H), 2. 05 (quint., 2H), 1.20 (t, 3H).*

### Beispiel 22: 1-(2-{3-[(4-Methyl-5-phenyl-4H-1,2,4-triazol-3-yl)thio]propyl}-2,3-dihydro-1H-isoindol-5-yl)propan-1-on Hydrochlorid

Analog zu Vorschrift 1.5 stellte man aus 4-Methyl-5-phenyl-4*H*-1,2,4-triazole-3-thiol (1.43mmol, 0.33g) und 1-[2-(3-Chloropropyl)-2,3-dihydro-1*H*-isoindol-5-yl]propan-1-on (1.43mmol, 0.36g; Herstellung analog zu 1.4 aus Isoindolin nach WO 0125200) die Titelverbindung her. Ausbeute: 0.24g.

*¹H-NMR (500 MHz, CDCl₃) δ ppm: 7.84-7.77 (m, 2H), 7.65-7.59 (m, 2H), 7.53-7.48 (m, 3H), 7.26 (d, 1H), 3.96 (s, 4H), 3.59 (s, 3H), 3.38 (t, 2H), 2.95 (q, 2H), 2.89 (t, 2H), 2.08 (quint., 2H), 1.20 (t, 3H).*

### Beispiel 23: 1-(2-{3-[(4-Methyl-5-pyridin-3-yl-4H-1,2,4-triazol-3-yl)thio]propyl}-2,3-dihydro-1H-isoindol-5-yl)propan-1-on

Analog zu Vorschrift 1.5 stellte man aus 4-Methyl-5-pyridin-3-yl-4*H*-1,2,4-triazol-3-thiol (1.43mmol, 0.33g) und 1-[2-(3-Chloropropyl)-2,3-dihydro-1*H*-isoindol-5-yl]propan-1-on (1.43mmol, 0.36g; ; Herstellung analog zu 1.4 aus Isoindolin nach WO 0125200) die Titelverbindung her. Ausbeute: 0.04g.

*¹H-NMR (500 MHz, CDCl₃) 6ppm: 8.87 (s br., 1H), 8. 70 (s br., 1H), 8. 02 (m, 1H), 7.87-7.75 (m, 1 H), 7.43 (m, 2H), 7. 25 (m, 1H), 4.02 (s, 4H), 3. 61 (s, 3H), 3.37 (t, 2H), 2.91 (m, 2H), 2.15 (t, 2H), 1.84 (m sym., 2H), 1.20 (t, 3H).*

### Beispiele für galenische Applikationsformen

### A) Tabletten

Auf einer Tablettenpresse werden in üblicher Weise Tabletten folgender Zusammensetzung gepresst:
40 mg Substanz des Beispiels 2
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6%iger Kleister)

### B) Dragees

20 mg Substanz des Beispiels 2
60 mg Kernmasse
70 mg Verzuckerungsmasse

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

Die zu testende Substanz wurde entweder in MethanoI/ChremophorO (BASF-AG) oder in Dimethylsulfoxid gelost und anschließend mit Wasser auf die gewünschte Konzentration verdünnt.

### Dopamin-D₃-Rezeptor:

Der Ansatz (0,250 ml) setzte sich zusammen aus Membranen von ∼10⁶ HEK-293 Zellen mit stabil exprimierten humanen Dopamin-D3-Rezeptoren, 0,1 nM [¹²⁵I]-Jodosulprid und Inkubationspuffer (totale Bindung) oder zusätzlich Testsubstanz (Hemmkurve) oder 1µM Spiperon (unspezifische Bindung). Dreifach-Ansätze wurden durchgeführt.

Der Inkubationspuffer enthielt 50 mM Tris, 120 mM NaC1, 5 mM KCI, 2 mM CaCl₂, 2 mM MgCl₂ und 0,1% Rinderserumalbumin, 10 µM Quinolone, 0.1 % Ascorbinsäure (täglich frisch hergestellt). Der Puffer wurde mit HCI auf pH 7.4 eingestellt.

### Dopamin-D₂(L)-Rezeptor:

Der Ansatz (1 ml) setzte sich zusammen aus Membranen von ∼ 10⁸ HEK-293 Zellen mit stabil exprimierten humanen Dopamin-D₂(L)-Rezeptoren (lange Isoform) sowie 0,01 nM [¹²⁵I]-Jodospiperon und Inkubationspuffer (totale Bindung) oder zusätzlich Testsubstanz (Hemmkurve) oder 1µM Haloperidol (unspezifische Bindung). Dreifach-Ansätze wurden durchgeführt.

Der Inkubationspuffer enthielt 50 mM Tris, 120 mM NaCl, 5 mM KCl, 2 mM CaCl₂, 2 mM MgCl₂ und 0,1 % Rinderserumalbumin. Der Puffer wurde mit HCl auf pH 7.4 eingestellt. Messung und Auswertung:
Nach Inkubation für 60 Minuten bei 25 °C wurden die Ansätze mit einem Zellsammelgerät über Wathman GF/B Glasfaserfilter unter Vakuum filtriert. Die Filter wurden mit einem Filter-Transfer-System in Szintillationsgläser überführt. Nach Zugabe von 4 ml Ultima Gold^{®} (Packard) wurden die Proben eine Stunde geschüttelt und anschließend die Radioaktivität im Beta-Counter (Packard, Tricarb 2000 oder 2200CA) gezählt. Die cp-Werte wurden anhand einer Standard-Quenchreihe mit Hilfe des geräteeigenen Programms in dpm umgerechnet.

Die Auswertung der Hemmkurven erfolgte durch iterative nichtlineare Regressionsanalyse mit dem Statistical Analysis System (SAS), ähnlich dem von Munson und Rodbard beschreibenen Programm "LIGAND".

Die erfindungsgemäßen Verbindungen zeigen in diesen Tests sehr gute Affinitäten am D₃-Rezeptor (< 100 nmolar insbesondere < 50 nM) und binden selektiv an den D₃-Rezeptor. Die Ergebnisse der Bindungstests sind in Tabelle 2 angegeben.

**Tabelle 2:**

| Verbindung Nr. | Ki (D₃) [nM] | Ki(D₂)/Ki(D₃) |
|---|---|---|
| Ia'-1 | 11,4 | 133 |
| Ia'-3 | 19,3 | 40 |
| Ia'-4 | 4,5 | 80 |
| Ia'-12 | 10,7 | 90 |
| Ia'-15 | 4,4 | 103 |
| Ia'-18 | 22,4 | 48 |
| Ia'-19 | 8,6 | 149 |
| Ia'-25 | 4,4 | 90 |
| Ia'-39 | 18,8 | 37 |
| Ia'-46 | 21,7 | 94 |
| Ia'-57 | 2,6 | 59 |
| Ia'-58 | 25,5 | 39 |
| Ia'-60 | 30,3 | 37 |
| Ia'-63 | 40,3 | 38 |
| Ia'-68 | 14,9 | 56 |
| Ia'-64 | 20,2 | 46 |
| Ib'-25 | 21,4 | 126 |
| Ib'-65 | 40,0 | 49 |

### Untersuchung der zerebralen Verfügbarkeit und der oralen Bioverfügbarkeit

Die Testsubstanzen wurden männlichen Wistarratten in Parallelversuchen jeweils intravenös (Schwanzvene, 2 mg/kg Körpergewicht) und oral (Schlundsonde, 10 mg/kg Körpergewicht) verabreicht. Zur intravenösen Verabreichung wurde die Testverbindung in Wasser mit bis zu 4 Vol-% DMSO und/oder Ethanol gelöst, zur oralen Verabreichung in 4 gew.-%iger, wässriger Hydroxypropylcellulose-Lösung suspendiert. Nach verschiedenen Zeitpunkten (intravenös: 5 min, 15 min, 30 min, 2 h, 8 h und 24 h; oral: 15 min, 30 min, 2 h, 8 h und 24 h) nach der Verabreichung wurden je 2 Ratten eine Blutprobe oder Himgewebe entnommen. In üblicher Weise wurde die Konzentration der Testverbindung im Blutplasma sowie im Himgewebe (Plasma und Hirn) durch gekoppelte Flüssigkeitschromatographie/Massenspektroskopie bzw. mittels HPLC bestimmt. Aus den erhaltenen Ergebnissen wurden die Fläche unter der Konzentrations-Zeit-Kurve für Him (AUC_{brain}) bzw. für Plasma (AUC_{IV}, AUCₒᵣₐₗ) anhand der Trapezmethode [((tₙ-tₙ₋₁)x(Cₙ+Cₙ₋₁)/2) berechnet, worin tₙ der Bestimmungszeitpunkt und tₙ₋₁ der vorhergehene Bestimmungszeitpunkt sind und Cₙ bzw. Cₙ₋₁ die Konzentrationen zum Zeitpunkt tₙ bzw. tₙ₋₁ sind]. Die maximale Konzentration der Wirkstoffe in Himgewebe cₘₐₓ brain, der Wert AUC_{brain} sowie das Verhältnis von AUC_{brain}/AUCₚₗₐₛₘₐ ist ein Maß für die Zerebralverffigbarkeit der Wirksubstanz. Ausserdem wurde in an sich bekannter Weise die Bioverfügbarkeit F des Wirkstoffs aus den Daten berechnet (AUCₒᵣₐₗ/AUC_{iV} in %). Die getesteten Wirkstoffe sowie die pharmakokinetischen Daten sind in Tabelle 3 angegeben. In der nachstehend angegebenen Formel haben Ar und R die in Tabelle 3 angegebenen Bedeutungen.

**Tabelle 3:**

| | | | | | | |
|---|---|---|---|---|---|---|
| 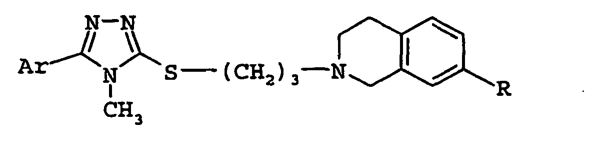 | | | | | | |

| Nr. | Ar | R | Cₘₐₓ (brain)* [ng/g] | AUC_{brain}* [ng h/g] | AUC_{brain}/AUCₚₗₐₛₘₐ | F [%] |
|---|---|---|---|---|---|---|
| A | 1-Methylpyrrol-2-yl | SO₂-Piperidinyl | u.d.N. | | n.b | 3 |
| B | 1-Methylpyrrol-2-yl | Methoxy | 87 | 228 | 2,1 | 3 |
| Ia'-15 | 1-Methylpyrrol-2-yl | C(O)-Ethyl | 686 | 2903 | 0,3 | 100 |
| Ia'-12 | 1-Methylpyrrol-2-yl | C(O)-Phenyl | 130 | 306 | 0,2 | 10 |

| | | | | | | |
|---|---|---|---|---|---|---|
| *aus oralen Versuchen, u.N.: unterhalb Nachweisgrenzen, n.b.: nicht bekannt, F: Bioverfügbarkeit A: Beispiel 35 der WO 00/42306 (freie Base) B: Beispiel 594 der WO 00/42306 (Hydrochlorid) Ia'-15 (Hydrochlorid) Ia'-12 (Hydrochlorid) | | | | | | |

Es zeigte sich, dass die erfindungsgemäßen Verbindungen eine deutlich höhere zerebrale Verfügbarkeit und Bioverfügbarkeit aufweisen als Vergleichsverbindungen, die nicht der Formel I entsprechen.

## Patentansprüche

1. Triazolverbindungen der allgemeinen Formel I, 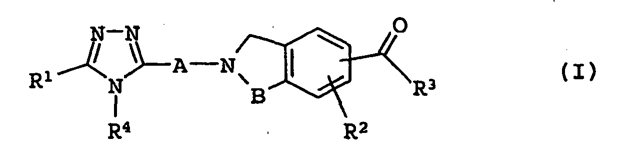 worin
A für C₄-C₁₀-Alkylen oder für C₃-C₁₀-Alkylen, das wenigstens eine Gruppe Z umfasst, die ausgewählt ist unter O, S, NR⁵, CONR⁵, COO, CO, wobei Alkylen auch eine C₃-C₆-Cycloalkylengruppe und/oder eine Doppel- oder Dreifachbindung aufweisen kann, und
B für CH₂ oder CH₂-CH₂ steht;
R¹ für einen aromatischen Rest steht, der ausgewählt ist unter Phenyl und einem 5-oder 6-gliedrigen heteroaromatischen Rest mit 1, 2, 3 oder 4 Heteroatomen, die unabhängig voneinander ausgewählt sind unter O, N und S, wobei der aromatische Rest einen oder mehrere Substituenten aufweisen kann, die unabhängig voneinander ausgewählt sind unter C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₃-C₆-Cycloalkyl, das gegebenenfalls durch Halogen oder C₁-C₄-Alkyl substituiert ist, Halogen, CN, OR⁶, COOR⁶, NR⁷R⁸, NO₂, SR⁹, SO₂R⁹, SO₂NR⁷R⁸, COR¹⁰, und Phenyl, das gegebenenfalls durch ein oder zwei Reste substituiert ist, die unabhängig voneinander ausgewählt sind unter C₁-C₄-Alkyl, C₁-C₄-Alkoxy, NR⁷R⁸, CN, CF₃, CHF₂ oder Halogen, wobei Phenyl und der heteroaromatische Rest auch mit einem 5 oder 6-gliedrigen, aromatischen oder nichtaromatischen Carbocyclus kondensiert sein kann;
R² H, C₁-C₆-Alkyl, das gegebenenfalls durch OH, C₁-C₄-Alkoxy, C₁-C₄-Alkylthio, Halogen oder Phenyl substituiert ist, OH, C₁-C₆-Alkoxy, OCF₃, OCHF₂, OSO₂CF₃, SH, C₁-C₆-Alkylthio, C₂-C₆-Alkenyl, C₂-C₆-Alkinyl, Halogen, CN oder NO₂, bedeutet;
R³ für C₂-C₁₀-Alkyl, C₁-C₆-Halogenalkyl, C₁-C₁₀-Alkyl, das durch C₁-C₄-Alkoxy, C₁-C₄-Alkylthio oder Phenyl substituiert ist, das seinerseits einen, zwei oder drei Substituenten, ausgewählt unter C₁-C₄-Alkyl, C₁-C₄-Halogenalkyl, CN, OR⁶, COOR⁶, NR⁷R⁸, NO₂, SR⁹, SO₂R⁹, SO₂NR⁷R⁸, COR¹⁰ und Halogen, aufweisen kann, für C₃-C₆-Cycloalkyl, das gegebenenfalls durch Halogen oder C₁-C₄-Alkyl substituiert ist, oder für einen aromatischen Rest steht, der ausgewählt ist unter Phenyl, Naphthyl und einem 5- oder 6-gliedrigen heteroaromatischen Rest mit 1, 2, oder 3 Heteroatomen, die unabhängig voneinander ausgewählt sind unter O, N und S, wobei der aromatische Rest einen oder zwei Substituenten aufweisen kann, die unabhängig voneinander ausgewählt sind unter C₁-C₈-Alkyl, C₁-C₆-Halogenalkyl, C₁-C₆-Alkoxy, CN, COOR⁶, NR⁷R⁸, NO₂, SO₂R⁹, SO₂NR⁷R⁸, COR¹⁰, CF₉, CHF₂ oder Halogen, wobei R³ auch für Methyl stehen kann, wenn R¹ für einen gegebenenfalls substituierten heteroaromatischen Rest steht;
R⁴ für H, C₁-C₆-Alkyl, das gegebenenfalls durch OH, C₁-C₄-Alkoxy oder Phenyl substituiert ist, C₁-C₆-Halogenalkyl, C₃-C₆-Cycloalkyl, das gegebenenfalls durch C₁-C₄-Alkyl, oder Halogen substituiert ist, oder Phenyl steht;
R⁵ für H, C₁-C₆-Alkyl, das gegebenenfalls durch OH, C₁-C₄-Alkoxy oder Phenyl substituiert ist, C₁-C₆-Halogenalkyl, Phenyl oder eine Gruppe COR¹¹ steht;
R⁶ bis R¹⁰ unabhängig voneinander für H, C₁-C₆-Alkyl, das gegebenenfalls durch OH, C₁-C₄-Alkoxy oder Phenyl substituiert ist, C₁-C₆-Halogenalkyl oder Phenyl, stehen, wobei R⁰ auch eine Gruppe COR¹¹ bedeuten kann, wobei R¹¹ eine der für R⁴ angegebenen Bedeutungen aufweist;
R⁷ mit R⁸ auch gemeinsam einen 5- oder 6-gliedrigen gesättigten oder ungesättigten Carbocyclus bilden kann, der ein Heteroatom, ausgewählt unter O, S, N und NR¹² als Ringglied aufweisen kann, wobei R¹² für Wasserstoff oder C₁-C₄-Alkyl steht;
und die physiologisch verträglichen Salze dieser Verbindungen.

2. Verbindungen nach Anspruch 1 der Formel I, worin B für CH₂CH₂ steht.

3. Verbindungen nach Anspruch 1 oder 2 der Formel I, worin A für C₄-C₁₀-Alkylen oder Z-C₃-C₁₀-Alkylen steht, wobei Alkylen eine Doppelbindung aufweisen kann, Z an den Triazol-Ring gebunden ist und ausgewählt ist unter O, S, COO, NR⁵ und CO.

4. Verbindungen nach Anspruch 3 der Formel I, worin A für eine Gruppe S-(C₃-C₁₀-Alkylen) steht, worin Alkylen eine Doppelbindung aufweisen kann.

5. Verbindungen nach einem der vorhergehenden Ansprüche der Formel I, worin R² für H steht.

6. Verbindungen nach einem der vorhergehenden Ansprüche der Formel I, worin R¹ für Phenyl steht, das unsubstituiert ist oder einen oder zwei Substituenten aufweist, die unabhängig voneinander ausgewählt sind unter C₁-C₈-Alkyl, OH, C₁-C₆-Alkoxy, Phenyl, CN und Halogen und/oder das mit einem 6-gliedrigen, aromatischen Carbocyclus kondensiert sein kann.

7. Verbindungen nach einem der Ansprüche 1 bis 5 der Formel I, worin R¹ für einen heteroaromatischen Rest steht, der ausgewählt ist unter Thienyl, Furanyl, Tetrazolyl, Pyrrolyl, Benzothienyl, Indolyl, Benzothiazolyl, Pyridyl oder Pyrazinyl, wobei der aromatische Rest in der in Anspruch 1 oder 6 angegebenen Weise substituiert sein kann.

8. Verbindungen nach Anspruch 7, worin R¹ für Pyrrolyl steht, das gegebenenfalls 1 oder 2 Substituenten ausgewählt unter C₁-C₄-Alkyl und C₃-C₈-Cycloalkyl aufweist.

9. Verbindungen nach einem der vorhergehenden Ansprüche der Formel I, worin R³ für C₂-C₁₀-Alkyl, C₃-C₆-Cycloalkyl, Benzyl oder Phenyl steht, das einen oder zwei Substituenten aufweisen kann, die unabhängig voneinander ausgewählt sind unter C₁-C₆-Alkyl, C₁-C₆-Alkoxy, CN, NO₂, CF₃, CHF₂ oder Halogen.

10. Verbindungen nach einem der vorhergehenden Ansprüche der Formel Ia, 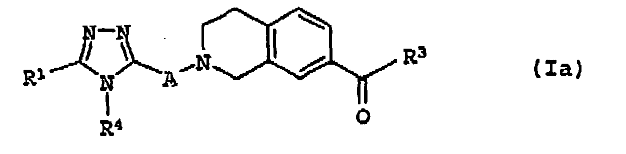 worin A, R¹ R³ und R⁴ die zuvor angegebenen Bedeutungen aufweisen.

11. Verbindungen nach Anspruch 10 der Formel Ia, worin
A für C₄-C₁₀-Alkylen oder Z-C₃-C₁₀-Alkylen steht, wobei Alkylen eine Doppelbindung aufweisen kann, Z an den Triazol-Ring gebunden ist und ausgewählt ist unter O, S, COO, CONR⁵, NR⁶ und CO;
R¹ für einen aromatischen Rest steht, der ausgewählt ist unter Phenyl, Thienyl, Furanyl, Tetrazolyl, Pyrrolyl, Benzothienyl, Indolyl, Benzothiazolyl, Pyridyl oder Pyrazinyl, wobei der aromatische Rest in der in Anspruch 1 angegebenen Weise substituiert sein kann;
R³ für C₂-C₁₀-Alkyl, C₃-C₆-Cycloalkyl, Benzyl oder Phenyl steht, das einen oder zwei Substituenten aufweisen kann, die unabhängig voneinander ausgewählt sind unter C₁-C₆-Alkyl, C₁-C₆-Alkoxy, CN, NO₂, CF₃, CHF₂ oder Halogen, wobei R³ auch Methyl bedeuten kann, wenn R¹ nicht für gegebenenfalls substituiertes Phenyl steht, und
R⁴ Wasserstoff, C₁-C₆-Alkyl oder C₃-C₆-Cycloalkyl bedeutet

12. Verbindungen nach Anspruch 10 oder 11, worin R¹ für pyrrolyl steht, das gegebenenfalls 1 oder 2 Substituenten ausgewählt unter C₁-C₄-Alkyl und C₃-C₆-Cycloalkyl aufweist.

13. Verbindungen nach einem der Ansprüche 10 bis 12 der Formel Ia, worin A für eine Gruppe S-(C₃-C₁₀-Alkylen) steht, worin Alkylen eine Doppelbindung aufweisen kann.

14. Verbindungen nach Anspruch 11, worin R² für Methyl steht, A eine Gruppe z'-(CH₂)ₖ bedeutet, wobei Z' an den Triazolring gebunden ist und Sauerstoff. Schwefel oder CH₂ bedeutet, k für 3 oder 4 steht,
R¹ Phenyl, 4-Fluorphenyl, Pyrrol-2-yl, 1-Methylpyrrol-2-yl, 2-Thienyl, 3-Thienyl, Benzothien-2-yl, 2-Furyl, 3-Furyl, Berzothiazol-2-yl, 2-Pyridinyl, 3-Pyridinyl, 4-Pyridinyl oder 2-Pyrazinyl bedeutet und
R³ für C₂-C₄-Alkyl oder Phenyl, das ein- oder zweifach durch C₁-C₄-Alkyl, C₁-C₄-Alkoxy, CF₃, CHF₂ oder Halogen substituiert sein kann, steht, oder wenn R¹ von Phenyl verschieden ist auch Methyl bedeuten kann.

15. Pharmazeutisches Mittel, enthaltend wenigstens eine Verbindung der Formel nach einem der Ansprüche 1 bis 14, gegebenenfalls zusammen mit physiologisch akzeptablen Trägem und/oder Hilfsstoffen.

16. Verwendung wenigstens einer Verbindung der Formel I nach einem der Ansprüche 1 bis 14 zur Herstellung eines pharmazeutischen Mittels zur Behandlung von Erkrankungen, die auf die Beeinflussung von Dopamin-D₃-Rezeptoren ansprechen.

17. Verwendung nach Anspruch 16 zur Behandlung von Störungen des zentralen Nervensystems.

## Claims

1. Triazole compounds of the formula I 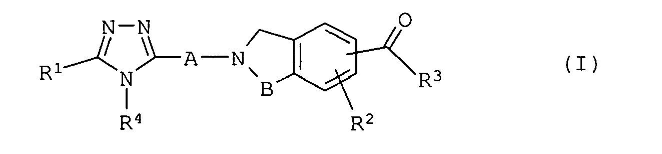 in which
A is C₄-C₁₀-alkylene or C₃-C₁₀-alkylene which includes at least one group Z which is selected from O , S, NR⁵, CONR⁵, COO and CO , where alkylene may also have a C₃-C₆-cycloalkylene group and/or a double or triple bond,
B is CH₂ or CH₂-CH₂;
R¹ is an aromatic radical which is selected from phenyl and a 5- or 6-membered heteroaromatic radical having 1, 2, 3 or 4 heteroatoms which are selected independently of one another from O, N and S, where the aromatic radical may have one or more substituents which are selected independently of one another from C₁-C₆-alkyl which is optionally substituted by OH, C₁-C₄-alkoxy, halogen or phenyl, or C₂-C₆-alkenyl, C₂-C₆-alkynyl, C₃-C₆-cycloalkyl which is optionally substituted by halogen or C₁-C₄-alkyl, or halogen, CN, OR⁶, COOR⁶, NR⁷R⁸, NO₂, SR⁹, SO₂R⁹, SO₂NR⁷R⁸, COR¹⁰, and phenyl which is optionally substituted by one or two radicals which are selected independently of one another from C₁-C₄-alkyl, C₁-C₄-alkoxy, NR⁷R⁸, CN, CF₃, CHF₂ or halogen, where phenyl and the heteroaromatic radical may also be fused to a 5 or 6-membered, aromatic or nonaromatic carbocycle;
R² is H, C₁-C₆-alkyl which is optionally substituted by OH, C₁-C₄-alkoxy, C₁-C₄-alkylthio, halogen or phenyl, or OH, C₁-C₆-alkoxy, OCF₃, OCHF₂, OSO₂CF₃, SH, C₁-C₆-alkylthio, C₂-C₆-alkenyl, C₂-C₆-alkynyl, halogen, CN or NO₂;
R³ is C₂-C₁₀-alkyl, C₁-C₆-haloalkyl, C₁-C₁₀-alkyl which is substituted by C₁-C₄-alkoxy, C₁-C₄-alkylthio or phenyl which may in turn have one, two or three substituents selected from C₁-C₄-alkyl, C₁-C₄-haloalkyl, CN, OR⁶, COOR⁶, NR⁷R⁸, NO₂, SR⁹, SO₂R⁹, SO₂NR⁷R⁸, COR¹⁰ and halogen, or is C₃-C₆-cycloalkyl which is optionally substituted by halogen or C₁-C₄-alkyl, or is an aromatic radical which is selected from phenyl, naphthyl and a 5- or 6-membered heteroaromatic radical having 1, 2, or 3 heteroatoms which are selected independently of one another from O, N and S, where the aromatic radical may have one or two substituents which are selected independently of one another from C₁-C₆-alkyl, C₁-C₆-haloalkyl, C₁-C₆-alkoxy, CN, COOR⁶, NR⁷R⁸, NO₂, SO₂R⁹, SO₂NR⁷R⁸, COR¹⁰, CF₃, CHF₂ or halogen, where R³ may also be methyl if R¹ is an optionally substituted heteroaromatic radical;
R⁴ is H, C₁-C₆-alkyl which is optionally substituted by OH, C₁-C₄-alkoxy or phenyl, or C₁-C₆-haloalkyl, C₃-C₆-cycloalkyl which is optionally substituted by C₁-C₄-alkyl or halogen, or phenyl;
R⁵ is H, C₁-C₆-alkyl which is optionally substituted by OH, C₁-C₄-alkoxy or phenyl, or C₁-C₆-haloalkyl, phenyl or a COR¹¹ group;
R⁶ to R¹⁰ are independently of one another H, C₁-C₆-alkyl which is optionally substituted by OH, C₁-C₄-alkoxy or phenyl, or C₁-C₆-haloalkyl or phenyl, where R⁸ may also be a COR¹¹ group in which R¹¹ has one of the meanings mentioned for R⁴;
R⁷ may also form together with R⁸ a 5- or 6-membered saturated or unsaturated carbocycle which may have a heteroatom selected from O, S, N and NR¹² as ring member, where R¹² is hydrogen or C₁-C₄-alkyl,
and the physiologically tolerated salts of these compounds.

2. Compounds according to Claim 1 of the formula I in which B is CH₂CH₂.

3. Compounds according to Claim 1 or 2 of the formula I in which A is C₄-C₁₀-alkylene or Z-C₃-C₁₀-alkylene, where alkylene may have a double bond, Z is bonded to the triazole ring and is selected from O , S, COO, NR⁵ and CO.

4. Compounds according to Claim 3 of the formula I in which A is a group S-(C₃-C₁₀-alkylene) in which alkylene may have a double bond.

5. Compounds according to any of the preceding claims of the formula I in which R² is H.

6. Compounds according to any of the preceding claims of the formula I in which R¹ is phenyl which is unsubstituted or has one or two substituents which are selected independently of one another from C₁-C₆-alkyl , OH, C₁-C₆-alkoxy, phenyl, CN and halogen and/or which may be fused to a 6-membered aromatic carbocyle.

7. Compounds according to any of Claims 1 to 5 of the formula I in which R¹ is a heteroaromatic radical which is selected from thienyl, furanyl, tetrazolyl, pyrrolyl, benzothienyl, indolyl, benzothiazolyl, pyridyl or pyrazinyl, where the aromatic radical may be substituted in the manner indicated in Claim 1 or 6.

8. Compounds according to Claim 7, in which R¹ is pyrrolyl which optionally has 1 or 2 substituents selected from C₁-C₄-alkyl and C₃-C₆-cycloalkyl.

9. Compounds according to any of the preceding claims of the formula I in which R³ is C₂-C₁₀-alkyl, C₃-C₆-cycloalkyl, benzyl or phenyl which may have one or two substituents which are selected independently of one another from C₁-C₆-alkyl, C₁-C₆-alkoxy, CN, NO₂, CF₃, CHF₂ or halogen.

10. Compounds according to any of the preceding claims of the formula Ia 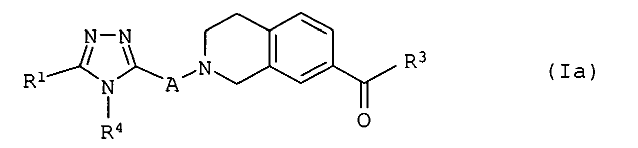 in which A*,* R¹ R³ and R⁴ have the meanings indicated above.

11. Compounds according to Claim 10 of the formula Ia in which
A is C₄-C₁₀-alkylene or Z-C₃-C₁₀-alkylene, where alkylene may have a double bond, Z is bonded to the triazole ring and is selected from O, S, COO, CONR⁵, NR⁵ and CO;
R¹ is an aromatic radical which is selected from phenyl, thienyl, furanyl, tetrazolyl, pyrrolyl, benzothienyl, indolyl, benzothiazolyl, pyridyl or pyrazinyl, where the aromatic radical may be substituted in the manner indicated in claim 1;
R³ is C₂-C₁₀-alkyl, C₃-C₆-cycloalkyl, benzyl or phenyl which may have one or two substituents which are selected independently of another from C₁-C₆-alkyl, C₁-C₆-alkoxy, CN, NO₂, CF₃, CHF₂ or halogen, where R³ may also be methyl if R¹ is not optionally substituted phenyl, and
R⁴ is hydrogen, C₁-C₆-alkyl or C₃-C₆-cycloalkyl.

12. Compounds according to Claim 10 or 11 in which R¹ is pyrrolyl which optionally has 1 or 2 substituents selected from C₁-C₄-alkyl and C₃-C₆-cycloalkyl.

13. Compounds according to any of Claims 10 to 12 of the formula Ia in which A is a group S-(C₃-C₁₀-alkylene) in which alkylene may have a double bond.

14. Compounds according to Claim 11 in which R² is methyl, A is a group Z'-(CH₂)ₖ where Z' is bonded to the triazole ring and is oxygen, sulfur or CH₂, k is 3 or 4,
R¹ is phenyl, 4-fluorophenyl, pyrrol-2-yl, 1-methylpyrrol-2-yl, 2-thienyl, 3-thienyl, benzothien-2-yl, 2-furyl, 3-furyl, benzothiazol-2-yl, 2-pyridinyl, 3-pyridinyl, 4-pyridinyl or 2-pyrazinyl, and
R³ is C₂-C₄-alkyl or phenyl which may be substituted once or twice by C₁-C₄-alkyl, C₁-C₄-alkoxy, CF₃, CHF₂ or halogen, or may also be methyl if R¹ is different from phenyl.

15. Pharmaceutical composition comprising at least one compound of the formula I according to any of Claims 1 to 14, where appropriate together with physiologically acceptable carriers and/or excipients.

16. Use of at least one compound of the formula I according to any of Claims 1 to 14 for producing a pharmaceutical composition for the treatment of disorders which respond to influencing of dopamine D₃ receptors.

17. Use according to Claim 16 for the treatment of disorders of the central nervous system.

## Revendications

1. Composés de triazole de formule générale (I), 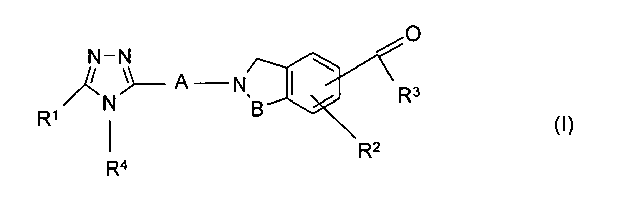 où
A représente C₄-C₁₀-alkylène ou C₃-C₁₀-alkylène, qui comprend au moins un groupe Z qui est choisi parmi O, S, NR⁶, CONR⁶, COO, CO, où alkylène peut aussi comporter un groupe C₃-C₆-cycloalkylène et/ou une double ou triple liaison, et
B représente CH₂ ou CH₂-CH₂ ;
R¹ représente un groupement aromatique qui est choisi parmi phényle et un groupement hétéroaromatique à 5 ou 6 chaînons ayant 1, 2, 3 ou 4 hétéroatomes qui sont choisis indépendamment les uns des autres parmi O, N et S, où le groupement aromatique peut comporter un ou plusieurs substituants qui sont choisis indépendamment les uns des autres parmi C₁-C₆-alkyle, qui est éventuellement substitué par OH, C₁-C₄-alcoxy, halogène ou phényle, C₂-C₆-alcényle, C₂-C₆-alcynyle, C₃-C₆-cycloalkyle qui est éventuellement substitué par halogène ou C₁-C₄-alkyle, halogène, CN, OR⁶, COOR⁶, NR⁷R⁸, NO₂, SR⁹, SO₂R⁹, SO₂NR⁷R⁸, COR¹⁰, et phényle, qui est éventuellement substitué par 1 ou 2 groupements qui sont choisis indépendamment l'un de l'autre parmi C₁-C₄-alkyle, C₁-C₄-alcoxy, NR⁷R⁸, CN, CF₃, CHF₂ ou halogène, où phényle et le groupement hétéroaromatique peuvent aussi être condensés avec un carbocycle aromatique ou non aromatique à 5 ou 6 chaînons ;
R² représente H, C₁-C₆-alkyle, qui est éventuellement substitué par OH, C₁-C₄-alcoxy, C₁-C₄-alkylthio, halogène ou phényle, OH, C₁-C₆-alcoxy, OCF₃, OCHF₂, OSO₂CF₃, SH, C₁-C₆-alkylthio, C₂-C₆-alcényle, C₂-C₆-alcynyle, halogène, CN ou NO₂ ;
R³ représente C₂-C₁₀-alkyle, C₁-C₆-halogénoalkyle, C₁-C₁₀-alkyle qui est substitué par C₁-C₄-alcoxy, C₁-C₄-alkylthio ou phényle, qui peut comporter pour sa part 1, 2 ou 3 substituants choisis parmi C₁-C₄-alkyle, C₁-C₄-halogénoalkyle, CN, OR⁶, COOR⁶, NR⁷R⁸, NO₂, SR⁹, SO₂R⁹, SO₂NR⁷R⁸, COR¹⁰ et halogène, C₃-C₆-cycloalkyle qui est éventuellement substitué par halogène ou C₁-C₄-alkyle ou un groupement aromatique qui est choisi parmi phényle, naphtyle et un groupement hétéroaromatique à 5 ou 6 chaînons ayant 1, 2 ou 3 hétéroatomes qui sont choisis indépendamment les uns des autres parmi O, N et S, où le groupement aromatique peut comporter 1 ou 2 substituants qui sont choisis indépendamment l'un de l'autre parmi C₁-C₆-alkyle, C₁-C₆-halogénoalkyle, C₁-C₆-alcoxy, CN, COOR⁶, NR⁷R⁸, NO₂, SO₂R⁹, SO₂NR⁷R⁸, COR¹⁰, CF₃, CHF₂ ou halogène, où R³ peut aussi représenter méthyle quand R¹ représente un groupement hétéroaromatique éventuellement substitué ;
R⁴ représente H, C₁-C₆-alkyle, qui est éventuellement substitué par OH, C₁-C₄-alcoxy ou phényle, C₁-C₆-halogénoalkyle, C₃-C₆-cycloalkyle qui est éventuellement substitué par C₁-C₄-alkyle ou halogène, ou phényle ;
R⁵ représente H, C₁-C₆-alkyle qui est éventuellement substitué par OH, C₁-C₄-alcoxy ou phényle, C₁-C₆-halogénoalkyle, phényle ou un groupe COR¹¹ ;
R⁶ à R¹⁰ représentent indépendamment les uns des autres H, C₁-C₆-alkyle, qui est éventuellement substitué par OH, C₁-C₄-alcoxy ou phényle, C₁-C₆-halogénoalkyle ou phényle, où R⁸ peut représenter aussi un groupe COR¹¹ où R¹¹ présente l'une des significations indiquées pour R⁴ ;
R⁷ peut aussi former avec R⁸ conjointement un carbocycle saturé ou insaturé à 5 ou 6 chaînons qui peut comporter un hétéroatome choisi parmi O, S, N et NR¹² comme membre du cycle, où R¹² représente l'hydrogène ou C₁-C₄-alkyle ;
et les sels physiologiquement acceptables de ces composés.

2. Composés selon la revendication 1 de formule I où B représente CH₂CH₂.

3. Composés selon la revendication 1 ou 2 de formule I où A représente C₄-C₁₀-alkylène ou Z-C₃-C₁₀-alkylène, où alkylène peut comporter une double liaison, Z est lié au cycle triazole et est choisi parmi O, S, COO, NR⁵ et CO.

4. Composés selon la revendication 3 de formule I, où A représente un groupe S-(C₃-C₁₀-alkylène), où alkylène peut comporter une double liaison.

5. Composés selon l'une des revendications précédentes de formule 1 où R² représente H.

6. Composés selon l'une des revendications précédentes de formule I, où R¹ représente phényle qui est non substitué ou comporte un ou deux substituants qui sont choisis indépendamment l'un de l'autre parmi C₁-C₆-alkyle, OH, C₁-C₆-alcoxy, phényle, CN et halogène et/ou qui peut être condensé avec un carbocycle aromatique à 6 chaînons.

7. Composés selon l'une des revendications 1 à 5 de formule I où R¹ représente un groupement hétéroaromatique qui est choisi parmi thiényle, furanyle, tétrazolyle, pyrrolyle, benzothiényle, indolyle, benzothiazolyle, pyridyle ou pyrazinyle, où le groupement aromatique peut être substitué de la manière indiquée dans la revendication 1 ou 6.

8. Composés selon la revendication 7, où R¹ représente pyrrolyle qui comporte éventuellement 1 ou 2 substituants choisis parmi C₁-C₄-alkyle et C₃-C₆-cyclalkyle.

9. Composés selon l'une des revendications précédentes de formule I, où R³ représente C₂-C₁₀-alkyle, C₃-C₆-cycloalkyle, benzyle ou phényle qui peut comporter un ou deux substituants qui sont choisis indépendamment l'un de l'autre parmi C₁-C₆-alkyle, C₁-C₆-alcoxy, CN, NO₂, CF₃, CHF₂ ou halogène.

10. Composés selon l'une des revendications précédentes de formule Ia : 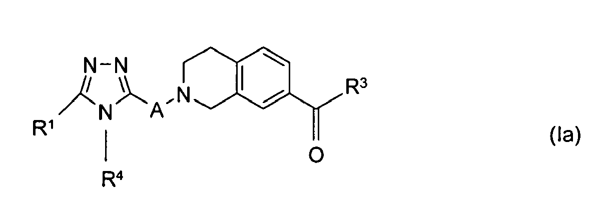 où A, R¹, R³ et R⁴ présentent les significations indiquées précédemment.

11. Composés selon la revendication 10 de formule Ia, où
A représente C₄-C₁₀-alkylène ou Z-C₃-C₁₀-alkylène, où alkylène peut comporter une double liaison, Z est lié au cycle triazole et est choisi parmi O, S, COO, CONR⁵, NR⁶ et CO ;
R¹ représente un groupement aromatique qui est choisi parmi phényle, thiényle, furanyle, tétrazolyle, pyrrolyle, benzothiényle, indolyle, benzothiazolyle, pyridyle ou pyrazinyle, où le groupement aromatique peut être substitué de la manière indiquée dans la revendication 1 ;
R³ représente C₂-C₁₀-alkyle, C₃-C₆-cycloalkyle, benzyle ou phényle, qui peut comporter un ou deux substituants qui sont choisis indépendamment l'un de l'autre parmi C₁-C₆-alkyle, C₁-C₆-alcoxy, CN, NO₂, CF₃, CHF₂ ou halogène, où R³ peut aussi représenter méthyle quand R¹ ne représente pas phényle éventuellement substitué, et
R⁴ représente l'hydrogène, C₁-C₆-alkyle ou C₃-C₆-cycloalkyle.

12. Composés selon la revendication 10 ou 11, où R¹ représente pyrrolyle qui comporte éventuellement 1 ou 2 substituants choisis parmi C₁-C₄-alkyle et C₃-C₆-cycloalkyle.

13. Composés selon l'une des revendications 10 à 12 de formule Ia, où A représente un groupe S-(C₃-C₁₀-alkylène) où alkylène peut comporter une double liaison.

14. Composés selon la revendication 11, où R² représente méthyle, A représente un groupe Z'-(CH₂)ₖ ou Z' est lié au cycle triazole et représente l'oxygène, le soufre ou CH₂, k représente 3 ou 4,
R¹ représente phényle, 4-fluorophényle, pyrrol-2-yle, 1-méthylpyrrol-2-yle, 2-thiényle, 3-thiényle, benzothién-2-yle, 2-furyle, 3-furyle, benzothiazol-2-yle, 2-pyridinyle, 3-pyridinyle, 4-pyridinyle ou 2-pyrazinyle, et
R³ représente C₂-C₄-alkyle ou phényle qui peut être substitué une ou deux fois par C₁-C₄-alkyle, C₁-C₄-alcoxy, CF₃, CHF₂ ou halogène, ou quand R¹ est différent de phényle peut représenter aussi méthyle.

15. Agent pharmaceutique contenant au moins un composé de formule I selon l'une des revendications 1 à 14 éventuellement en même temps que des vecteurs et/ou adjuvants physiologiquement acceptables.

16. Utilisation d'au moins un composé de formule I selon l'une des revendications 1 à 14 pour la fabrication d'un agent pharmaceutique pour le traitement des maladies qui réagissent à une influence sur les récepteurs de dopamine D₃.

17. Utilisation selon la revendication 16 pour le traitement des troubles du système nerveux central.
